# EUROPEAN PATENT SPECIFICATION

(11) **EP 1 984 030 B1**
(45) Date of publication and mention of the grant of the patent: **08.05.2013**
(21) Application number: 07762648.9
(22) Date of filing: 30.01.2007
(51) Int. Cl.: G01N 33/574

(54) **METHODS FOR DETECTION OF CIRCULATING TUMOR CELLS AND METHODS OF DIAGNOSIS OF CANCER IN A MAMMALIAN SUBJECT**
VERFAHREN FÜR DEN NACHWEIS ZIRKULIERENDER TUMORZELLEN UND VERFAHREN ZUR DIAGNOSTIZIERUNG VON KREBS BEI EINEM SÄUGER
PROCEDES DE DETECTION DE CELLULES TUMORALES CIRCULANTES ET PROCEDES DE DIAGNOSTIC DE CANCER CHEZ UN SUJET MAMMALIEN

(30) Priority: 30.01.2006 US 763625 P
(43) Date of publication of application: 29.10.2008
(73) Proprietor: THE SCRIPPS RESEARCH INSTITUTE, La Jolla, CA 92037 (US)
(72) Inventor: KUHN, Peter, Solana Beach, CA 92075 (US); MARRINUCCI, Dena, Solana Beach, CA 92075 (US)
(74) Representative: Fisher, Adrian John
(86) International application number: PCT/US2007/002798
(87) International publication number: WO 2007/089911

(56) References cited:
- US-A1- 2003 129 676
- US-B2- 6 670 197
- D. MARRINUCCI ET AL: "Case study of the morphologic variation of circulating tumor cells" HUMAN PATHOLOGY, vol. 38, no. 3, 13 February 2007 (2007-02-13), pages 514-519, XP005881889 SAUNDERS, PHILADELPHIA, PA, USA ISSN: 0046-8177
- H.B. HSIEH ET AL: "High speed detection of circulating tumor cells" BIOSENSORS & BIOELECTRONICS, vol. 21, no. 10, 15 April 2006 (2006-04-15), pages 1893-1899, XP024961663 ELSEVIER SCIENCE PUBLISHERS, BARKING, GB ISSN: 0956-5663 [retrieved on 2006-04-15]
- KRIVACIC ET AL.: 'A rare cell detector for cancer' PNAS vol. 101, no. 29, 20 July 2004, pages 10501 - 10504, XP002432922
- CURRY ET AL.: 'High Speed Detection of occult tumor cells in peripheral blood' PROCEEDINGS OF THE 26TH ANNUAL INTERNATIONAL CONFERENCE OF THE IEEE EMBS, SAN FRANCISCO, CA, USA 01 September 2004 - 05 September 2004, XP010775174
- G. VONA ET AL.: "Isolation by size of epithelial tumor cells", AMERICAN JOURNAL OF PATHOLOGY, vol. 156, no. 1, 1 January 2000 (2000-01-01), pages 57-63, USA

## Description

### FIELD OF INVENTION

The disclosure generally relates to a method for detecting circulating epithelial tumor cells in a mammalian subject and to methods of diagnosing cancer in a mammalian subject. The methods of detection or diagnosis indicate the presence of metastatic cancer or an early stage cancer.

### BACKGROUND

Circulating epithelial tumor cells (CTCs) have been observed in the peripheral blood of patients with epithelial-derived cancers at ultra low concentrations of 1 in 10⁶ to 10⁷ peripheral blood mononuclear cells. Kraeft et al., Clin Cancer Res 10: 3020-3028, 2004; Kraeft et al., Methods Mol Med 75: 423-430, 2003; Meng et al., Clin Cancer Res 10: 8152-8162,2004; Witzig et al., Clin Cancer Res 8: 1085-1091, 2002; Pantel et al., Curr Opin Oncol 12: 95-101, 2000; Gross et al., Proc Natl Acad Sci USA 92: 537-541, 1995. The number of these cells has been shown to correlate with outcome for cohorts of metastatic breast cancer patients with progressive disease at the time of sampling. Cristofanilli et al., N Engl J Med351: 781-91, 2004. Their characterization is, hence, of considerable biomedical interest in order to understand how these cells can travel via the blood stream to anatomically distant sites and form metastatic disease. Consequently, an instrument for measuring these cells could be a valuable diagnostic tool.

Hematogenous metastasis is the major cause of mortality in breast cancer, contributing to an estimated 41,000 deaths in the United States in 2005. Jemal et al. Cancer J Clin 55: 10-30, 2005. Evidence indicates that tumor cells are shed from the primary tumor mass into the bloodstream. Tarin, Cancer Metastasis Rev 1: 215-225, 1982; Tarin et aL, Cancer Res 41: 3604-3609, 1981; Klein et al., Proc Natl Acad Sci USA 96: 4494-4499, 1999; Liotta et al., Cell 64: 327-336, 1991. The factors involved in CTC survival in the blood and eventual metastasis are not well understood. Chambers et al., Breast Cancer Res 2: 400-407, 2000. CTCs observed in the peripheral blood of patients with epithelial-derived cancers can be identified using immunofluorescence assays via monoclonal antibodies directed against cytokeratins (CK), or other epithelial cell specific markers. Moll, Subcell Biochem 31: 205-262, 1998; Pantel et al., J Hemalother 3: 165-173, 1994. Prospective clinical trials have established that these CK+ cells are malignant and their presence in blood is correlated with poor outcome and lower survival rate in a number of studies. Fehm et al., Clin Cancer Res 8: 2073-2084, 2002. Cristofanilli et al., N Engl J Med 351: 781-791, 2004; Cristofanilli et al., J Clin Oncol 23: 1420-1430, 2005; Pierga et al., Clin Cancer Res 10: 1392-1400, 2004.

The most reliable method currently available for CTC detection is automated digital microscopy (ADM) using image analysis for recognition of immunocytochemically labeled tumor cells. ADM, however, is disadvantaged by its very slow scan speeds of 800 cells/sec. Kraeft et al., Clin Cancer Res 10: 3020-8, 2004. The ADM scan speed is constrained by the latency associated with stepping the sample many times due to the limited field of view.

To circumvent this speed constraint, several CTC enrichment technologies have been developed to reduce the total number of cells that need scanning. Hager et al., Gynecol Oncol 98: 211-6, 2005; Rosenberg et al., Cytometry 49: 150-8, 2002; Witzig et al., Clin Cancer Res 8: 1085-91, 2002; Umiel et al., J Hematother Stem Cell Res 9: 895-904, 2000; Siewert et al., Recent Results Cancer Res 158: 51-60, 2001; US 6, 670, 197 and Vona et al., American Journal of Pathology, 156, 1, January 2000. To date the most successful of these enrichment approaches is immunomagnetic enrichment (IME). Smimov et al., Cancer Res 65: 4993-7, 2005; Allard et al., Clin Cancer Res 10: 6897-904, 2004; Cristofanilli et al., N Engl JMed 351: 781-91, 2004; US 2003/129676. In most implementations of IME, monoclonal antibodies conjugated to small magnetic beads target the epithelial cell adhesion molecule, EpCAM. The beads are then manipulated in magnetic fields for enrichment. However, expression levels of EpCAM in CTCs are known to be substantially reduced from the levels of cells in tissues. Rao et al., Int J Oncol 27: 49-57, 2005. Since sensitivity loss was observed in enrichment of cells with reduced EpCAM expression, this approach could have low sensitivity for some CTCs. Krivacic et al., Proc Natl Acad Sci USA 101: 10501-4,2004.

Motivated by the limitations of available technology, a scanning instrument has been developed using fiber-optic array scanning technology (FAST) that can locate CTCs at a rate that is 500-times faster than ADM with comparable sensitivity and improved specificity and, consequently, does not require an enrichment step. Krivacic et al., Proc Natl Acad Sci USA 101: 10501-4, 2004; Curry et al., Proc. of the 26th annual international conference of the IEEE EMBS, San Francisco, CA, USA 01 September 2004-05 September 2004. The scanning instrument includes a light collection system that has a very large field of view (50 mm) with no loss of collection efficiency. This wide collection aperture (100-fold increase over ADM) is implemented with a thin wide bundle of optical filters that collect the fluorescent emission. This field-of-view is large enough to enable continuous scanning and eliminate the need to step the sample, which is the main source of latency.

Currently, CTCs are detected and analyzed primarily through immunocytochemical markers such as CK and the use of nuclear staining with DAPI. Although these approaches have been successful in enumerating and distinguishing CTCs, they differ from standard cytopathologic approaches as they omit the correlation with standard morphologic staining upon which diagnostic pathology is dependent. Cristofanilli et al., N Engl J Med 351: 781-791, 2004; Fehm et al., Cytotherapy 7: 171-185, 2005. This creates difficulty in comparing CTCs to tumor cells from other sites obtained by routine diagnostic procedures. Although the ability to detect CTCs has the potential to aide in diagnostic and individualized treatment of cancer and efficacy of treatment, the understanding of the biology of CTCs could be improved by including standard cytopathologic methods. A need exists in the art to utilize detailed high resolution imaging of CTCs with conventional diagnostic pathology staining methods and bright-field microscopy to confer the potential to make a standard cytopathologic diagnosis of circulating carcinoma and advance the adoption of diagnosis using CTCs in the clinic.

### SUMMARY

The invention provides a method for detecting circulating epithelial tumor cells in a mammalian subject suspected of having cancer comprising:
(i) mounting a test sample on a substrate, the test sample being from blood of the subject, the test sample comprising a cell population which has not been enriched for circulating tumor cells;
(ii) detecting the presence or absence of a first marker in the test sample, wherein the first marker is specific for circulating epithelial tumor cells and is a cytokeratin marker;
(iii) detecting the presence or absence of a second marker in the cells of the cell population or a subset of the cell population, wherein the second marker is cell morphology, cell size, or nuclear to cytoplasmic ratio, and is detected with a cytological stain; and
(iv) analyzing the cell population detected by the first and second markers to identify circulating epithelial tumor cells.

The invention also provides a method of screening a drug candidate compound for treatment of epithelial cancer in a mammalian subject, said method comprising:
(i) mounting first and second test samples on a substrate, the first sample being a sample from blood of a subject suspected of having cancer before treatment of the subject with the drug candidate compound, and the second test sample being a sample from blood of the subject after treatment of the subject with the drug candidate compound, the test samples comprising a cell population which has not been enriched for circulating tumor cells;
(ii) detecting the presence or absence of a first marker in the test samples wherein the first marker is specific for circulating epithelial tumor cells and is a cytokeratin marker;
(iii) detecting the presence or absence of a second marker in the cells of the cell population or a subset of the cell population, wherein the second marker is cell morphology, cell size, or nuclear to cytoplasmic ratio, and is detected with a cytological stain; and
(iv) analyzing the cell population detected by the first and second markers to identify circulating epithelial tumor cells in the test samples before treatment with the drug candidate compound compared to after treatment with the drug candidate compound; wherein the presence of a decreased number of the circulating epithelial tumor cells in the sample after treatment compared to a number of the circulating epithelial tumor cells in the sample before treatment indicating effectiveness of the drug candidate compound in treating the cancer in the mammalian subject.

The present disclosure generally relates to a method for detecting circulating epithelial tumor cells (CTCs) in a mammalian subject or a method of diagnosing cancer in a mammalian subject. The present disclosure further provides a method of screening a drug candidate compound in a mammalian subject as a treatment of cancer. The cancer can be a metastatic cancer or an early stage cancer. The present disclosure further provides an apparatus for use in a method for detecting circulating tumor cells to provide point-of-care patient screening, monitoring and management of metastatic cancer or an early stage cancer.

A method for detecting circulating epithelial tumor cells in a mammalian subject suspected of having cancer is disclosed comprising obtaining a test sample from blood of the mammalian subject, the test sample comprising a cell population, mounting the test sample on a substrate, detecting a first marker in the test sample that selectively binds to the circulating tumor cells, detecting a second marker in the test sample that binds to the cell population or a subset of the cell population, and analyzing the cell population detected by the first and second markers to identify and characterize the circulating tumor cells. In one aspect of the method, the presence of the circulating tumor cells in the specimen indicates presence of metastatic cancer in the mammalian subject. In a further aspect, presence of the circulating tumor cells in the specimen indicates presence of an early stage cancer in the mammalian subject, or the presence or absence of the circulating tumor cells in the specimen indicates presence of a disease free state or a non-measurable disease state in the mammalian subject. In a further aspect, presence or absence of the circulating tumor cells in the specimen monitors therapy management during cancer therapy or cancer recovery. In a detailed aspect, the cell population is a mixed cell population. The substrate can be a planar substrate.

In one aspect of the method, mounting the test sample on the planar substrate forms a biological monolayer. The first marker or the second marker can be a fluorescent marker. In a further aspect, the first marker selectively binds to epithelial cells. In a detailed aspect, the first marker is a cytokeratin marker. The second marker can be a cytologic stain to identify the circulating tumor cell by morphology, size, or nuclear to cytoplasmic ratio. The cytologic stain can be Wright-Giemsa stain. The first marker or the second marker can be a cell-specific marker. In a detailed aspect, the cell-specific marker is cytokeratin, CD45, M30, chemokine receptor, CXCR1, CXCR4, CD44, CD24, VEGF, EGFR, or HuR. The test sample can further comprise a cell population sorted by a third marker. A further aspect of the method comprises sorting the cell population prior to mounting the test sample on the planar substrate.

The method for detecting circulating tumor cells in the mammalian subject further comprises analyzing the cell population by nuclear detail, nuclear contour, presence or absence of nucleoli, quality of cytoplasm, or quantity of cytoplasm. The method can further comprise analyzing the cell population by measuring intact cells with a high nuclear to cytoplasmic ratio, intact cells with a low nuclear to cytoplasmic ratio, early apoptotic cells, or late apoptotic cells, and identifying the circulating tumor cells. In another aspect of the method, detecting the first marker further comprises analyzing the cell population by cell attachment to the substrate, scanning the cell population on the substrate by fiber optic array, and imaging the cells by digital microscopy using relocation. In the method, detecting the second marker further comprises relocating epithelial cells identified by the first marker by digital microscopy. In a detailed aspect, the presence of the circulating tumor cells in the specimen indicates presence of cancer including, but not limited to, lymphoma, myeloma, neuroblastoma, breast cancer, ovarian cancer, lung cancer, rhabdomyosarcoma, small-cell lung tumors, primary brain tumors, stomach cancer, colon cancer, pancreatic cancer, urinary bladder cancer, testicular cancer, lymphomas, thyroid cancer, neuroblastoma, esophageal cancer, genitourinary tract cancer, cervical cancer, endometrial cancer, adrenal cortical cancer, or prostate cancer.

A method of diagnosing cancer in a mammalian subject suspected of having cancer is disclosed wherein the method comprises obtaining a test sample from blood of the subject, the test sample comprising a cell population, mounting the test sample on a substrate, detecting a first marker in the test sample that selectively binds to the circulating tumor cells, detecting a second marker in the test sample that binds to the cell population or a subset of the cell population, and analyzing the cell population detected by the first and second markers to identify and characterize the circulating tumor cells. In one aspect of the method, the presence of the circulating tumor cells in the specimen indicates presence of metastatic cancer in the mammalian subject. In a further aspect, presence of the circulating tumor cells in the specimen indicates presence of an early stage cancer in the mammalian subject, or the presence or absence of the circulating tumor cells in the specimen indicates presence of a disease free state or a non-measurable disease state in the mammalian subject. In a further aspect, presence or absence of the circulating tumor cells in the specimen monitors therapy management during cancer therapy or cancer recovery. In a detailed aspect, the cell population is a mixed cell population. The substrate can be a planar substrate.

In one aspect of the method, mounting the test sample on the substrate forms a biological monolayer. The first marker or the second marker can be a fluorescent marker. In a further aspect, the first marker selectively binds to epithelial cells. In a detailed aspect, the first marker is a cytokeratin marker. The second marker can be a cytologic stain to identify the circulating tumor cell by morphology, size, or nuclear to cytoplasmic ratio. The cytologic stain can be Wright-Giemsa stain. The first marker or the second marker can be a cell-specific marker. In a detailed aspect, the cell-specific marker is cytokeratin, CD45, M30, chemokine receptor, CXCR1, CXCR4, CD44, CD24, VEGF, EGFR, or HuR. The test sample can further comprise a cell population sorted by a third marker. A further aspect of the method comprises sorting the cell population prior to mounting the test sample on the substrate. The cell population can be sorted by a number of methods known in the art, for example, by red blood cell lysis or by sorting the cells for a cell marker. Cell sorting for a cell marker can occur as a positive selection for circulating tumor cells or as a negative selection to remove non-tumor cells.

The method for diagnosing cancer in the mammalian subject further comprises analyzing the cell population by nuclear detail, nuclear contour, presence or absence of nucleoli, quality of cytoplasm, or quantity of cytoplasm. The method can further comprise analyzing the cell population by measuring intact cells with a high nuclear to cytoplasmic ratio, intact cells with a low nuclear to cytoplasmic ratio, early apoptotic cells, or late apoptotic cells, and identifying the circulating tumor cells. In another aspect of the method, detecting the first marker further comprises analyzing the cell population by cell attachment to the substrate, scanning the cell population on the substrate by fiber optic array, and imaging the cells by digital microscopy using relocation. In the method, detecting the second marker further comprises relocating epithelial cells identified by the first marker by digital microscopy. In a detailed aspect, the presence of the circulating tumor cells in the specimen indicates presence of cancer including, but not limited to, lymphoma, myeloma, neuroblastoma, breast cancer, ovarian cancer, lung cancer, rhabdomyosarcoma, small-cell lung tumors, primary brain tumors, stomach cancer, colon cancer, pancreatic cancer, urinary bladder cancer, testicular cancer, lymphomas, thyroid cancer, neuroblastoma, esophageal cancer, genitourinary tract cancer, cervical cancer, endometrial cancer, adrenal cortical cancer, or prostate cancer.

In one aspect of the method, the presence of the circulating epithelial tumor cells in the specimen indicating the likelihood of cancer recurrence in the mammalian subject. In a further aspect of the method the presence of the circulating tumor cells in the specimen indicating the cancer remission status in the mammalian subject.

A method of screening a drug candidate compound for treatment of cancer in a mammalian subject is disclosed wherein the method comprises administering a therapeutically effective amount of the drug candidate compound to the mammalian subject suspected of having cancer, obtaining test samples from blood of the subject before and after treatment with the drug candidate compound, the test samples comprising a cell population suspected of containing circulating tumor cells, mounting the test samples on a substrate, detecting a first marker in the test samples that selectively binds to the circulating tumor cells, detecting a second marker in the test samples that binds to the cell population or a subset of the cell population, and analyzing the cell population detected by the first and second markers to identify the circulating tumor cells in the test samples before treatment with the drug candidate compound compared to after treatment with the drug candidate compound, wherein the presence of a decreased number of the circulating tumor cells in the specimen after treatment compared to a number of the circulating tumor cells in a specimen before treatment indicating effectiveness of the drug candidate compound in treating the cancer in the mammalian subject. The cancer can be metastatic cancer or an early stage cancer. The cell population can be a mixed cell population. The substrate can be a planar substrate.

In one aspect of the method, mounting the test sample on the substrate forms a biological monolayer. The first marker or the second marker can be a fluorescent marker. In a further aspect, the first marker selectively binds to epithelial cells. In a detailed aspect, the first marker is a cytokeratin marker. The second marker can be a cytologic stain to identify the circulating tumor cell by morphology, size, or nuclear to cytoplasmic ratio. The cytologic stain can be Wright-Giemsa stain. The first marker or the second marker can be a cell-specific marker. In a detailed aspect, the cell-specific marker is cytokeratin, CD45, M30, chemokine receptor, CXCR1, CXCR4, CD44, CD24, VEGF, EGFR, or HuR. The test sample can further comprise a cell population sorted by a third marker. A further aspect of the method comprises sorting the cell population prior to mounting the test sample on the substrate. The cell population can be sorted by a number of methods known in the art, for example, by red blood cell lysis or by sorting the cells for a cell marker. Cell sorting for a cell marker can occur as a positive selection for circulating tumor cells or as a negative selection to remove non-tumor cells.

The type of cancer includes, but is not limited to, lymphoma, myeloma, neuroblastoma, breast cancer, ovarian cancer, lung cancer, rhabdomyosarcoma, small-cell lung tumors, primary brain tumors, stomach cancer, colon cancer, pancreatic cancer, urinary bladder cancer, testicular cancer, lymphomas, thyroid cancer, neuroblastoma, esophageal cancer, genitourinary tract cancer, cervical cancer, endometrial cancer, adrenal cortical cancer, or prostate cancer.

### BRIEF DESCRIPTION OF THE DRAWINGS

Figure 1 shows fiber-optic array scanning technology (FAST).

Figure 2 shows an example of pathologist identified circulating tumor cell (CTC) found in the peripheral blood of a breast cancer patient (20x).

Figure 3 shows a plot of total intensity of objects identified by FAST for a cancer patient and for HT-29 cells on a separate substrate used to monitor the sample preparation.

Figure 4 shows Wright-Giemsa stain and a fluorescent image of a typical CTC found in breast cancer patients.

Figure 5 shows an example of pathologist identified CTC found in the peripheral blood of a breast cancer patient (60x) and a corresponding Wright-Giemsa stain of the same CTC (100x).

Figure 6 shows a spectrum of fluorescent and Wright-Giemsa stained CTCs identified from a patient.

Figure 7 shows a probable CTC undergoing final stage of apoptosis.

Figures 8A-8D show histologic and cytologic features of a primary and metastatic tumor in a patient.

Figure 9 shows apoptotic CTCs verified as CK⁺ caspase-3⁺ found in a breast cancer patient.

Figure 10 shows status and CTC counts of patients with progressive disease, as determined by radiographic and/or clinical findings were more likely to have CTCs detected in peripheral blood by tandem FAST-ADM.

Figure 11 shows overall survival rate of breast cancer patients based on tandem FAST-ADM CTC determination.

### DETAILED DESCRIPTION

The present disclosure is generally related to a method for detecting circulating epithelial tumor cells (CTCs) in a mammalian subject or a method of diagnosing metastatic cancer or an early stage cancer in a mammalian subject. The present disclosure further relates to a method of screening a drug candidate compound in a mammalian subject for treatment of metastatic cancer. The method for detecting CTCs in the mammalian subject is disclosed which comprises obtaining from a tissue of the mammalian subject suspected of having cancer, a blood specimen comprising a mixed cell population suspected of containing CTC_{S}, mounting the blood cells and CTCs on a substrate to form a biological monolayer, detecting in the biological monlayer a first marker that selectively binds to the CTCs, detecting in the biological monolayer a second marker that binds to the mixed cell population or a subset of the mixed cell population, analyzing the cell population detected by the first marker and the second marker to identify CTCs, the presence of the CTCs in the specimen indicating the presence of metastatic cancer or early stage cancer in the mammalian subject. The presence or absence of the CTCs in the specimen can indicate the presence of a disease free state or a non-measurable disease state in the mammalian subject.

Although not in accordance with the present invention, the method could provide a cell attachment protocol to identify epithelial-derived cells within a blood sample, in conjunction with fiber array scanning technology (FAST) to detect CTCs in blood of cancer patients. In this protocol, live white blood cells (WBCs) e.g., leukocytes, and other cells in the blood are isolated on a slide, for example, as a biological monolayer. Leukocytes include, but are not limited to: T-lymphocytes; monocytes, eosinophils, and neutrophils, involved in phagocytosis; basophils involved in inflammatory response. In a method of the present invention; WBCs and CTCs are attached on specially coated adhesive slides and immunofluorescently labeled with a cytokeratin (CK) antibody cocktail on the slide surface after fixation and permeabilization. As normal cells of the blood and bone marrow are mesenchymal in origin, they do not express CK proteins, making the disseminated epithelial cells readily distinguishable from normal blood cells. The slides are then analyzed by FAST and the coordinates of the rare cells on each slide are obtained.

Methods utilizing FAST and the cell attachment protocol can be used to investigate the prevalence ofCTCs in metastatic breast and lung cancer patients. An additional advantage of the method enables a pathologist to relocate and examine cells of interest for pathologic confirmation and characterization. In the present invention, the protocol further includes removing the coverslip and/or solubilizing the water-soluble mounting media on each fluorescently stained slide and re-staining the same cells using a second cell marker, e.g., a standard Wright-Giemsa staining, to provide additional insights into CTC morphology, size, and heterogeneity. Known CK⁺ individual rare cells and rare cell clusters which were located by FAST and the cell attachment protocol can be evaluated morphologically. Although fluorescent images ofCTCs have aided in their verified identification, the Wright-Giemsa stain has provided additional cytologic information about CTCs. In a further aspect of the disclosure, the method can be used to evaluate different cell markers that are specific for either a disease, disease state, cell type, or cell state.

The ability to detect and characterize CTCs has the potential to aide in the diagnostic and individualized treatment of cancer patients. Due to their rarity, special methods are required to investigate CTCs. The present invention provides an approach that enables the use of standard cytopathologic methods for detailed morphologic characterization ofCTCs in blood obtained from breast cancer patients and provides details of cytologic characteristics of a spectrum of CTCs. Nucleated cells recovered from whole blood are deposited onto adhesive slides, immunofluorescently labeled, and analyzed with fiber-optic array scanning technology for detection of CTCs. Coupling these techniques with routine staining methods enables identification and evaluation of CTCs using light microscopy. Using conventional pathologic methods to observe the cells, CTCs exhibit a high degree of inter- and intra-patient pleomorphism in whole blood preparations, and intact CTCs are identified with both high and low nuclear-to-cytoplasmic ratios along with CTCs exhibiting apoptotic hallmarks. Morphologic observations suggest that the full spectrum of cells present in primary and metastatic tumor sites may also be seen circulating in blood, and furthermore provide a possible framework of morphologic classification within which to investigate the properties of cell subsets involved in metastasis.

A cell attachment protocol using indirect immunofluorescence for epithelial-derived cells used in conjunction with fiber-optic array scanning technology (FAST) for detection of CTCs has been validated for identification of CTCs. Krivacic el al., Proc Natl Acad Sci USA 101: 10501-10504, 2004. The FAST system has established its potential usefulness for detection of CTCs in the peripheral blood of metastatic breast cancer patients. The present invention provides the results of this approach in tandem with diagnostic pathology staining methods, e.g., Geimsa staining or fluorescent staining, and light microscopy to study the heterogeneity of CTCs and compare the results with analysis of primary and metastatic tumor tissue. This approach enables high quality verification of CTCs from blood obtained from cancer patients and has facilitated in the creation of an image gallery that provides detailed morphologic and cytologic characteristics of a spectrum of CTCs.

### AUTOMATED DIGITAL MICROSCOPY AND FIBER-OPTIC ARRAY SCANNING TECHNOLOGY

Methods are provided to detect epithelial-derived tumor cells which circulate in peripheral blood at ultra-low concentrations in cancer patients. Although not in accordance with the present invention, an instrument could be developed capable of rapid and accurate detection of rare cells in circulation utilizing fiber-optic array scanning technology (FAST). The FAST cytometer can locate immunofluorescently labeled rare cells on glass substrates at scan rates 500 times faster than conventional automated digital microscopy. These high scan rates are achieved by collecting fluorescent emissions using a fiber bundle with a large (50mm) field of view. Very high scan rates make possible the ability to detect rare events without the requirement for an enrichment step. The FAST cytometer was used to detect, image, and re-image CTCs in peripheral blood of breast cancer patients. This technology has the potential to serve as a clinically useful point-of-care diagnostic and a prognostic tool for cancer clinicians. The use of a fixed substrate permits the re-identification and re-staining of cells allowing for additional morphologic and biologic information to be obtained from previously collected and identified cells.

FAST technology has been used for high speed detection of CTCs in peripheral blood of stage IV breast cancer patients. FAST scanning enables efficient imaging of CTCs with ADM so that 10 ml of blood containing about 60 million white blood cells can be evaluated in 80 minutes. Technology improvements that should enable this scan time to be reduced by over 75%. High resolution ADM images are further used for CTC identification. The results support using this instrument for point-of-care patient screening, monitoring and management. Finally, the ability to relocate cells enables the morphological and potentially the molecular characterization of CTCs, thus demonstrating its potential value for research of metastasis.

Automated digital microscopy (ADM) in combination with fiber-optic array scanning technology (FAST) is a reliable method for detection of cancer cells in blood and an important tool for diagnosis and monitoring of solid tumors in early stages. The preferred method of detection, ADM, is too slow to scan the large substrate areas. An approach that uses FAST applies laser-printing techniques to the rare-cell detection problem. With FAST cytometry, laser-printing optics are used to excite 300,000 cells per second, and emission is collected in an extremely wide field of view, enabling a 500-fold speed-up over ADM with comparable sensitivity and superior specificity. The combination of FAST enrichment and ADM imaging has the performance required for reliable detection of early-stage cancer in blood. Krivacic et al., Proc. Natl. Acad Sci. USA 101: 10501-10504, 2004.

It is estimated that CTCs are present in circulation at concentrations between 10⁻⁶ and 10⁻⁷. Pantel and Otte, Semin. Cancer Biol. 11: 327-237, 2001. Assuming the lower end of this range, 10⁻⁷, a sample of at least 100 million hematopoietic cells is needed to detect at least one CTC with a high probability (99.995%). ADM analysis of such a sample size would take 18 hours, resulting in 3,000-30,000 objects for a cytopathology examination. Borgen, et al., Cytometry 46: 215-221, 2001; Bauer, et al., Clin. Cancer Res. 6: 3552-2559, 2000; Kraeft, et al., Clin. Cancer Res. 6: 434-442, 2000; Mehes, et al., Cytometry 42: 357-362, 2000. Based on the performance using ADM and FAST, a FAST prescan of 100 million cells would take 5 minutes and result in 1,500 objects for subsequent rescanning by ADM. With the improved specificity of the tandem approach, this rescanning with ADM would require subsequent manual examination of only 300 objects. With the tandem approach, the task of screening 100 million hematopoietic cells could be completed within 1 hour.

*Automated Digital Microscopy.* Coordinates of prospective cells identified by the FAST cytometer could be fed into the rare-event imaging system (REIS), a fully automated scanning digital microscopy system. The hardware components of the REIS and the proprietary scanning software have been described in detail elsewhere. Krivacic et al., Proc. Natl. Acad Sci. USA 101: 10501-10504, 2004; Kraeft et al., Clin. Cancer Res. 6: 434-442, 2060; Kraeft et al., Clin. Cancer Res. 10: 3020-3028, 2004.

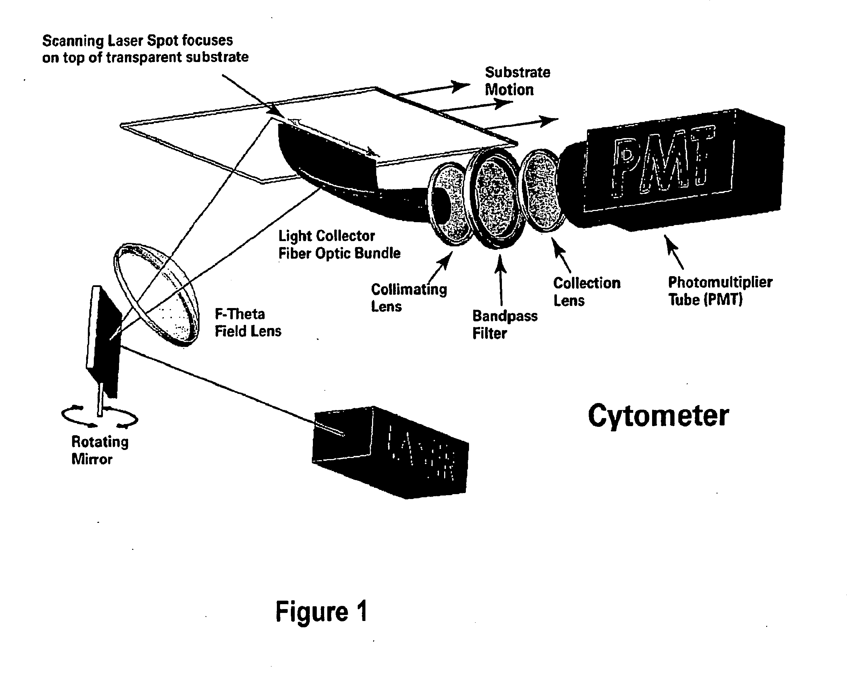

*Optical System.* The large field of view could be enabled by an optical fiber bundle with asymmetric ends. As shown in Figure 1, the collection end is long (50 mm) and narrow (2 mm), whereas the transmission end is circular (11.3 mm in diameter). In operation, an argon ion laser scans the substrate lying on top of the collection end, and the collected emission is subsequently collimated after the circular aperture. The emission from the fluorescent probes is filtered by using standard dichroic filters before detection in a photomultiplier. The sample moves across the laser scan path on a stage traveling in a direction orthogonal to the laser scan direction. The location of a fluorescing cell is determined accurately by the scan and stage positions at the time of emission. The scanning mechanics enable accurate determination of the emission location (better than 100 µm) for subsequent reviewing. Krivacic et al., Proc. Natl. Acad. Sci. USA 101: 10501-10504, 2004.

The laser is scanned at 100 scans per sec by using a galvanometer-rotated mirror, whereas the substrate is moved at 2 mm·sec⁻¹ over it, which produces an exposure rate of 1 cm²·sec⁻¹. The galvanometer can operate at this scan velocity over a 25° scan angle with linear angular time response. An F-Theta field lens transforms the 15.2° actual mirror deflection into linear displacement with a transformation accuracy better than 0.1%, which results in a reproducible distortion over the field of <82 µm that can be eliminated in the software. The 10-µm-diameter focused beam is transformed into an elliptical spot size, 10 × 20 µm, by the 60° angle of incidence. The ellipse major axis, which is normal to the sweep direction, defines the pixel resolution along one dimension. The sweep rate of 10 m·sec⁻¹ defines the 1-µsec transit time per pixel.

The intrinsic detection threshold of the FAST cytometer is currently determined by the autofluorescence from the borosilicate in the fiber optics and collimation lenses that is stimulated by scattered laser light. For 50 mW of laser power incident on a quartz substrate, this autofluorescence is ≈15 times the combination of all the other noise sources (such as noise in the electronics) and corresponds to the equivalent emission from ≈1,000 fluorescein molecules. For OTC detection, however, the measured autofluorescence from blood exceeds this intrinsic autofluorescence by 10-fold, and consequently, it is the autofluorescence from the blood that determines the effective detection threshold for this application.

A comparison of detection threshold between FAST and digital microscopy in the presence of blood autofluorescence was made by using a calibration slide containing a dilution series of Alexa Fluor 488 dye in rows differing in concentration by a factor of 2. Both instruments scanned the slides, and the signal strength for each row was compared with the strength of the autofluorescence coming from a blood sample under identical scanning conditions. In the presence of background autofluorescence, FAST can detect cell fluorescence that is approximately eight times dimmer than can be detected with a digital microscope. This difference can be attributed to a combination of more efficient excitation of the Alexa Fluor 488 probe by the 488-nm laser and more efficient excitation of the blood autofluorescence by the microscope broadband mercury light source. This detection advantage enables FAST to detect cells with lower levels offluorescence and, hence, lower levels of expressed target protein.

*Measurements.* Detected fluorescent objects are analyzed with software filter operations to differentiate rare cells from false positives. Because the cells are generally smaller than the laser-spot resolution (20 µm), the first filter passes all objects that are below a size threshold (20 µm). A second filter analyzes the ratio between the intensities of the fluorescence from different channels to eliminate homogeneous dye aggregates, a common artifact of immunofluorescence staining.

In one aspect of the invention the method for detecting circulating epithelial tumor cells in a mammalian subject or the method of diagnosing metastatic cancer in a mammalian subject utilizes an apparatus for imaging a generally planar surface. See U.S. Application Nos. 2004/0071330 and 2004/0071332. Although not in accordance with the present invention, in a further aspect, the method could utilize an imager apparatus capable of rapid and acuurate detection of rare cells in circulation utilizing fiber-optic array scanning technology (FAST). An imager apparatus for imaging a generally planar surface could be used. A linearly translating stage linearly translates the surface in a first direction. A fiber optic bundle has a first end of parallel first fiber ends arranged to define a linear input aperture disposed perpendicular to the first direction and parallel to the surface. The fiber optic bundle further has a second end defining a generally circular output aperture. Each first fiber end optically communicates with the generally circular output aperture. A scanning radiation source linearly scans a radiation beam along the generally planar surface below the input aperture. The radiation beam interacts with the surface to produce a light signal that is collected by the input aperture and transmitted by the fiber optic bundle to the output aperture. A photodetector is arranged to detect the light signal at the generally circular output aperture. A rastering processor communicates with the imager stage and the scanning radiation source to coordinate the scanning of the radiation beam and the linear translation of the surface to effectuate a rastering of the radiation beam on the surface.

A sample can be prepared as a biological monolayer by drawing a sample of a biological fluid including, but not limited to, blood or parts of blood from a subject. In one aspect, the sample is a monolayer of cells. The fluid sample is treated with a fluorescent material, such as but not limited to a marker dye, that selectively bonds to different kinds of biological molecules, which may be on the surface or inside the cell, such as proteins, nucleic acids or other molecules. Suitable markers are known in the art for marking a number of different cell types of clinical interest, including selected cancer cell types, fetal cells, or other appropriate cells to be considered. Markers for numerous other cells such as brain cells, liver cells, as well as bacteria cells, among others can be developed. The material emits a characteristic output, such as fluorescence or phosphorescence, responsive to a selected excitation irradiation, such as irradiation by a selected wavelength or spectrum of light, x-ray irradiation, electron-beam irradiation, or the like. The characteristic luminescence typically has a characteristic wavelength or spectral range of wavelengths. While dyes are the predominant tagging process, other techniques exist including the use of markers known as quantum dots and DNA nano-particle probes.

Although not in accordance with the present invention, in another aspect, an apparatus and method could be used for identifying rare cells in a biological monolayer. See, for example, U.S. Application No. 2004/0071332 . The rare cells on the biological monolayer emit a characteristic luminescence responsive to exposure to an excitation radiation. A translating stage is able to translate the biological monolayer in a first and a second direction. A fiber-optic bundle includes a plurality of fibers each having a first end and a second end. The first ends are arranged to define a generally rectangular receiving aperture having a large aspect ratio whose long dimension is perpendicular to the first direction. The second ends are arranged to define an output aperture having a compact shape. A radiation source linearly sweeps an excitation radiation beam across the first portion of the biological monolayer with a sweep direction perpendicular to the first direction. An interaction region of the radiation source and the first portion of the biological monolayer is arranged relative to the receiving aperture such that characteristic luminescence produced in the interaction region is collected by the receiving aperture. A photodetector is arranged to detect the collected characteristic luminescence at the output aperture. A controller controls the translation of the imager stage and the sweeping of the radiation source to raster the excitation radiation beam across the first portion of the biological monolayer to identify rare cells in the first portion of the biological monolayer based upon the characteristic luminescence detected during the rastering. The controller further controls translation of the translation stage in a second direction to place a second portion of the biological monolayer in a position where the radiation source linearly sweeps the excitation radiation beam across the second portion of the biological monolayer with a sweep direction perpendicular to the first direction. An interaction region of the radiation source and the second portion of the biological monolayer are arranged relative to the receiving aperture such that characteristic luminescence produced in the interaction region is collected by the receiving aperture. The photodetector is arranged to detect the collected characteristic luminescence at the output aperture of the second portion.

Although not in accordance with the present invention, in another aspect a method for obtaining a position of a rare cell, e.g., a circulating tumor cell (CTC), within a biological monolayer could be devised. See, for example, U.S. Application No. 2004/0131241. A slide which carries at least one rare cell and has reticle marks arranged at positions which form substantially a right angle, is positioned in a slide holder of a first imaging system. A first coordinate space of the imaging system is defined, and coordinates of the reticle marks in the first coordinate space are designated. A second coordinate space of a second imaging system is defined, and the coordinates of the reticle marks in the second coordinate space is designated. Using the designated coordinates of the reticle marks of the first coordinate space, the coordinate conversion parameters are computed. Thereafter, coordinates of at least one object in the first coordinate space are designated, and the first coordinate space coordinates of the object are converted into unique coordinates in a second coordinate space, using the coordinate conversion parameters.

Once the rare cell or CTC has been localized the coverslip on the biological monolayer can be removed or the water-soluble mounting media can be solubilized on each fluorescently stained slide. The same cells can be re-stained using a second cell marker, e.g., standard Wright-Giemsa staining to provide insights into CTC morphology, size, and heterogeneity. Known cytokeratin positive (CK⁺) individual rare cells and rare cell clusters can be located and evaluated morphologically. Although fluorescent images ofCTCs have aided in their verified identification, the Wright-Giemsa stain has provided additional information about CTCs

Although not in accordance with the present invention, in a further aspect, this process could be used to evaluate different cell markers that are specific for either a disease, disease state or cell type, cell state. Methods of the present invention will aid in characterization of CTCs. It enables high quality verification of CTCs from blood obtained from cancer patients without enrichment, and provides insights into morphology and characteristics of CTCs.

The search for rare metastatic CTCs suggests that many CTCs are apoptotic and incapable of forming metastases and estimates that only 1 disseminated cancer cell in 10,000 can even establish a metastasis. Thus, detection, morphologic classification, and molecular characterization of these rare cells could target novel and directed therapies, demonstrating the clinical significance of CTCs.

It is to be understood that this invention is not limited to particular methods, reagents, compounds, compositions or biological systems, which can, of course, vary. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a cell" includes a combination of two or more cells, and the like.

The term "about" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

"Biological monolayer" refers to a blood sample which may exist in various states of cell separation or purification. For example, the biological monolayer can be partially purified and contain mononuclear cells and other cells after lysis of red blood cell has occurred.

"Sorting the cell population prior to mounting the test sample on a substrate" refers to removing a subset of the cell population from the test sample, e.g., the blood sample. Sorting can occur by selective cell lysis and centrifugation of a subfraction of cells. Sorting can also occur using a fluorescent cell marker and fluorescence activated cell sorting. Cell sorting for a cell marker can occur as a positive selection for circulating tumor cells or as a negative selection to remove non-tumor cells.

The "substrate" holds the test sample, e.g., a blood sample containing cells mounted for detection and analysis. In one aspect, the substrate can be planar. In a further aspect, the substrate can have some curvature. The substrate can be scanned by the FAST system and the circulating tumor cells located by digital microscopy.

"Subject", "mammalian subject" or "patient" refers to any mammalian patient or subject to which the methods of the invention can be applied. "Mammal" or "mammalian" refers to human patients and non-human primates, as well as experimental animals such as rabbits, rats, and mice, and other animals. In an exemplary embodiment, of the present invention, to identify subject patients for treatment according to the methods of the invention, accepted screening methods are employed to determine risk factors associated with a targeted or suspected disease or condition, e.g., cancer, or to determine the status of an existing disease or condition in a subject. These screening methods include, for example, conventional work-ups to determine risk factors that can be associated with the targeted or suspected disease or condition. These and other routine methods allow the clinician to select patients in need of therapy using the methods and formulations of the invention.

"Cancer", "malignancy", "solid tumor" or "hyperproliferative disorder" are used as synonymous terms and refer to any of a number of diseases that are characterized by uncontrolled, abnormal proliferation of cells, the ability of affected cells to spread locally or through the bloodstream and lymphatic system to other parts of the body (*i.e*., metastasize) as well as any of a number of characteristic structural and/or molecular features. A "cancerous" or "malignant cell" or "solid tumor cell" is understood as a cell having specific structural properties, lacking differentiation and being capable of invasion and metastasis. "Cancer" refers to all types of cancer or neoplasm or malignant tumors found in mammals, including carcinomas and sarcomas. Examples are cancers of the breast, lung, non-small cell lung, stomach, brain, head and neck, medulloblastoma, bone, liver, colon, genitourinary, bladder, urinary, kidney, testes, uterus, ovary, cervix, prostate, melanoma, mesothelioma, sarcoma,. (see DeVita, et al., (eds.), 2001, Cancer Principles and Practice of Oncology, 6th. Ed., Lippincott Williams & Wilkins, Philadelphia, PA ). "Hyperproliferative disease" refers to any disease or disorder in which the cells proliferate more rapidly than normal tissue growth. Thus, a hyperproliferating cell is a cell that is proliferating more rapidly than normal cells.

"Cancer-associated" refers to the relationship ofa nucleic acid and its expression, or lack thereof, or a protein and its level or activity, or lack thereof, to the onset of malignancy in a subject cell. For example, cancer can be associated with expression of a particular gene that is not expressed, or is expressed at a lower level, in a normal healthy cell. Conversely, a cancer-associated gene can be one that is not expressed in a malignant cell (or in a cell undergoing transformation), or is expressed at a lower level in the malignant cell than it is expressed in a normal healthy cell.

In the context of the cancer, the term "transformation" refers to the change that a normal cell undergoes as it becomes malignant. In eukaryotes, the term "transformation" can be used to describe the conversion of normal cells to malignant cells in cell culture.

"Proliferating cells" are those which are actively undergoing cell division and growing exponentially. "Loss of cell proliferation control" refers to the property of cells that have lost the cell cycle controls that normally ensure appropriate restriction of cell division. Cells that have lost such controls proliferate at a faster than normal rate, without stimulatory signals, and do not respond to inhibitory signals.

"Advanced cancer" means cancer that is no longer localized to the primary tumor site, or a cancer that is Stage III or IV according to the American Joint Committee on Cancer (AJCC).

"Well tolerated" refers to the absence of adverse changes in health status that occur as a result of the treatment and would affect treatment decisions.

"Metastatic" refers to tumor cells, e.g., human solid tumor or genitourinary malignancy, that are able to establish secondary tumor lesions in the lungs, liver, bone or brain of immune deficient mice upon injection into the mammary fat pad and/or the circulation of the immune deficient mouse.

"Early stage cancer" refers to tumor cells that have not spread from the primary site origin of the tumor to other sites in the body, or refers to non-metastatic tumor cells.

A "first marker" and a "second marker" identify a circulating tumor cell by cytological stain or by a cell specific marker. Cytological stains include, but are not limited to, Wright-Giemsa stain, or other cytological stains known in the art. See for example, B.F. Atkinson, Atlas of Diagnostic Cytopathology. 2nd Edition, W.B. Saunders Company, Ed., 2003. Cell specific markers include, but are not limited to, markers for cytokeratin, CD45, M30, chemokine receptor, CXCR1, CXCR4, CD44, CD24, vascular endothelial growth factor (VEGF), epithelial growth factor receptor (EGFR), or mRNA stability factor HuR. These markers identify various cell types, including cells of hematopoietic origin, cytokeratins on epithelial cells, breast cancer cells, prostate cancer cells, CD44, cell surface receptor recognizing hyaluronic acid, chemokine receptors, such as CXCR1 or CXCR4.

"Sorting" in the context of cells as used herein to refers to both physical sorting of the cells, as can be accomplished using, e.g., a fluorescence activated cell sorter, as well as to analysis of cells based on expression of cell surface markers, e.g., FACS analysis in the absence of sorting.

"Analyzing the cell population by nuclear detail, nuclear contour, presence or absence of nucleoli, quality of cytoplasm, or quantity of cytoplasm" and "analyzing the cell population by measuring intact cells with a high nuclear to cytoplasmic ratio, intact cells with a low nuclear to cytoplasmic ratio, early apoptotic cells, or late apoptotic cells, and identifying the circulating tumor cells" can occur utilizing techniques and analytical methods as described in B.F. Atkinson, Atlas of Diagnostic Cytopathology. 2nd Edition, W.B. Saunders Company, Ed., 2003., and B.F. Atkinson and J.F. Silverman, Atlas of Difficult Diagnoses in Cytopathology, 1st Edition, W.B. Saunders Company; 1998.

"Management of cancer therapy or cancer recovery" refers to *in vivo* or *in vitro* diagnostic tests to determine the stage of cancer progression or the effectiveness of a particular cancer therapy treatment.

### CANCER TREATMENT (Although this is not in accordance with the present invention).

A "solid tumor" includes, but is not limited to, sarcoma, melanoma, carcinoma, or other solid tumor cancer.

"Sarcoma" refers to a tumor which is made up of a substance like the embryonic connective tissue and is generally composed of closely packed cells embedded in a fibrillar or homogeneous substance. Sarcomas include, but are not limited to, chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma ofB cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

"Melanoma" refers to a tumor arising from the melanocytic system of the skin and other organs. Melanomas include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

"Carcinoma" refers to a malignant new growth made up of epithelial cells tending to infiltrate the surrounding tissues and give rise to metastases. Exemplary carcinomas include, for example, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma ex ulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypemephroid carcinoma; infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidernoid carcinoma, carcinoma mucosum, mucous carcinoma, carcinoma myxomatodes, naspharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signetring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tuberous carcinoma, verrucous carcinoma, and carcinoma viflosum.

"Leukemia" refers to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease--acute or chronic; (2) the type of cell involved; myeloid (myelogenous), lymphoid (lymphogenous), or monocytic; and (3) the increase or non-increase in the number of abnormal cells in the blood--leukemic or aleukemic (subleukemic). Leukemia includes, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

Additional cancers include, for example, Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalemia, cervical cancer, endometrial cancer, adrenal cortical cancer, and prostate cancer.

### DETECTABLE LABEL

The particular label or detectable group used in the assay can be detectable by spectroscopic, photochemical, biochemical, immunochemical, electrical, optical or chemical means. The particular type of label is not a critical aspect of the invention, so long as it does not significantly interfere with the specific binding of an antibody to the cellular marker on the cell or the circulating tumor cell used in the assay. The detectable group can be any material having a detectable physical or chemical property. Such detectable labels have been well-developed in the field of assays or immunoassays and, in general, most any label useful in such methods can be applied to the present invention. Thus, a label is any composition detectable by spectroscopic, photochemical, biochemical, immunochemical, electrical, optical or chemical means. Useful labels in the present invention include magnetic beads (*e.g.* Dynabeads™), fluorescent dyes (*e.g.,* fluorescein isothiocyanate, Texas red, rhodamine, and the like), radiolabels (*e.g.,* ³H, ¹⁴C, ³⁵S, ¹²⁵I, ¹²¹I, ¹¹²In, ⁹⁹mTc), other imaging agents such as microbubbles (for ultrasound imaging), ¹⁸F, ¹¹C, ¹⁵O, (for Positron emission tomography), ^{99m}TC, ¹¹¹In (for Single photon emission tomography), enzymes (e.g., horse radish peroxidase, alkaline phosphatase and others commonly used in an ELISA), and calorimetric labels such as colloidal gold or colored glass or plastic (*e.g.* polystyrene, polypropylene, latex, and the like) beads. Patents that described the use of such labels include U.S. Pat. Nos. 3,817,837; 3,850,752; 3,939,350; 3,996,345; 4,277,437; 4,275,149; and 4,366,241. See also Handbook of Fluorescent Probes and Research Chemicals (6th Ed., Molecular Probes, Inc., Eugene OR.).

The label can be coupled directly or indirectly to the desired component of the assay according to methods well known in the art. As indicated above, a wide variety of labels can be used, with the choice of label depending on sensitivity required, ease of conjugation with the compound, stability requirements, available instrumentation, and disposal provisions.

Non-radioactive labels are often attached by indirect means. Generally, a ligand molecule (e.g., biotin) is covalently bound to the molecule. The ligand then binds to an anti-ligand (e.g., streptavidin) molecule which is either inherently detectable or covalently bound to a signal system, such as a detectable enzyme, a fluorescent compound, or a chemiluminescent compound. A number of ligands and anti-ligands can be used. Where a ligand has a natural anti-ligand, for example, biotin, thyroxine, and cortisol, it can be used in conjunction with the labeled, naturally occurring anti-ligands. Alternatively, any haptenic or antigenic compound can be used in combination with an antibody.

The molecules can also be conjugated directly to signal generating compounds, e.g., by conjugation with an enzyme or fluorophore. Enzymes of interest as labels will primarily be hydrolases, particularly phosphatases, esterases and glycosidases, or oxidoreductases, particularly peroxidases. Fluorescent compounds include fluorescein and its derivatives, rhodamine and its derivatives, dansyl, umbelliferone, and the like Chemiluminescent compounds include luciferin, and 2,3-dihydrophthalazinediones, *e.g.,* luminol. For a review of various labeling or signal producing systems which can be used, see, U.S. Pat. No. 4,391,904.

Means of detecting labels are well known to those of skill in the art. Thus, for example, where the label is a radioactive label, means for detection include a scintillation counter or photographic film as in autoradiography. Where the label is a fluorescent label, it can be detected by exciting the fluorochrome with the appropriate wavelength of light and detecting the resulting fluorescence. The fluorescence can be detected visually, by means of photographic film, by the use of electronic detectors such as charge coupled devices (CCDs) or photomultipliers and the like. Similarly, enzymatic labels can be detected by providing the appropriate substrates for the enzyme and detecting the resulting reaction product. Finally simple calorimetric labels can be detected simply by observing the color associated with the label. Thus, in various dipstick assays, conjugated gold often appears pink, while various conjugated beads appear the color of the bead.

Some assay formats do not require the use of labeled components. For instance, agglutination assays can be used to detect the presence of the target antibodies. In this case, antigen-coated particles are agglutinated by samples comprising the target antibodies. In this format, none of the components need be labeled and the presence of the target antibody is detected by simple visual inspection.

Frequently, the cellular marker and antibodies to the cellular marker will be labeled by joining, either covalently or non-covalently, a substance which provides for a detectable signal.

Other embodiments and uses will be apparent to one skilled in the art in light of the present disclosures.

### EXEMPLARY EMBODIMENTS

### EXAMPLE 1

### FAST System, Automated Digital Microscopy and Circulating Tumor Cell Identification

*Sample Preparation: Cell Attachment and Immunofluorescent Labelling of Patient Samples.* Blood samples are processed on large substrates (10.8 x 7.6 cm with an active area of 9.5x4.5 cm) using a modified version of a previously described protocol. Kraeft et al., Methods Mol Med 75: 423-30, 2003. Briefly, blood anti-coagulated with ethylenediaminetetraacetic acid (EDTA) is subjected to lysis with isotonic ammonium chloride buffer (155 mM NH₄Cl, 10 mM KHCO₃, 0.1. mM EDTA, pH 7.4) at room temperature for 5 minutes. After centrifugation, the remaining mononuclear cell pellet is washed, re-suspended in phosphate buffered saline (PBS), and the total number of living peripheral blood mononuclear cells are attached to custom designed adhesive substrates (Paul Marienfeld GmbH & Co., KG, Bad Mergentheim, Germany). The cells are incubated for 40 minutes at 37° C in PBS and then culture media (45% Dulbecco's Modified Eagle's Medium (DMEM), 45% RPMI) containing serum proteins (10% fetal bovine serum) is added to promote attachment, and incubation continues for another 20 minutes. The deposited cells are fixed in 2% paraformaldehyde (pH 7.2-7.4) for 20 minutes, rinsed twice in PBS, and then further fixed and permeablized in ice-cold methanol for 5 minutes, rinsed in PBS and blocked with 20% human AB serum (Nabi Diagnostics, Boca Raton, FL) in PBS at 37°C for 20 minutes. Substrates are then incubated at 37° C for 1 hour with a monoclonal anti-pan cytokeratin antibody (H-1388, Sigma, St. Louis, MO) which recognizes human cytokeratins 1, 4, 5, 6, 8, 10, 13, 18, and 19. Subsequently, substrates are washed in PBS, incubated with a mixture of Alexa Fluor 488 and Alexa Fluor 555 conjugated goat anti-mouse antibody (A-21121 and A-21425, Molecular Probes, Eugene, OR) at 37° C for 30 minutes. Nuclear counterstaining is done with 0.5 µg/ml 4',6-diamidino-2-phenylindole (DAPI) (D-21490, Molecular Probes) in PBS at room temperature for 20 minutes. Substrates are mounted in an aqueous mounting medium (20mM tris pH 8.0, 0.5% n-propyl gallate and 90% glycerol) and left in the dark overnight for mounting medium to dry before the edges are sealed with nail polish.

*FAST System.* The FAST scanner scans samples at a rate of 25M cells main⁻¹ with a sensitivity of 98% and a specificity of 10⁻⁵. (Krivacic et al., Proc Natl Acad Sci USA 101: 10501-10504, 2004. The scan rate (100 lines sec-1) results from a fast laser raster. A 10m W Argon ion laser excites fluorescence in labeled cells that is collected in optics with a large (50mm) field-of-view. This field-of-view is enabled by an optical fiber bundle with asymmetric ends as shown in Figure 1. The fiber bundle has a numerical aperture of 0.66. The resolution of the scanning system (12 µm) is determined by the spot size of the scanning laser. The emission from the fluorescent probes is filtered using standard dichroic filters before detection in a photomultiplier. A laser scan speed of 10m/sec is accomplished with a galvanometer controlled scanning mirror. The sample is moved orthogonally across the laser scan path on a microscope stage at a rate of 3mm sec⁻¹. The location of a fluorescently labeled cell is determined by the scan and stage positions at the time of emission to an accuracy of +/- 70 µm. The emission detection threshold of the FAST optical system is comparable to the ADM that images FAST-identified objects and is described later. Determination of the FAST sensitivity and specificity using a cell line model sample as well as details of the FAST optical system are described elsewhere. Kraeft et al., Clin Cancer Res 10: 3020-8, 2004.

*Microscope:* The automated digital microscope (ADM) is a Nikon (Melville , NY) TE2000U fluorescent inverted microscope. A 20X Plan Fluor (extra long working distance, NA=0.45) microscope objective is used for the initial image acquisition and a 40X Plan Fluor (extra long working distance, NA=0.6) objective is used for acquiring images for cell analysis. The working distances for the 20X and 40X objectives are respectively 7.4 mm and 2.7-3.7 mm (correction collar). The field-of-view diameters through these objectives are 1.1 mm and 0.55 mm, although real image sizes are limited by the imaging CCD. The microscope has automated excitation and emission filter wheels (Lamda 10-2, Sutter Instrument, Novalto, CA) with Triple Band Filter Set for DAPI, Fluorescein Isothiocyanate (FITC), and Tetramethyl Rhodamine IsoThiocyanate (TRITC) (61000V2, Chroma Technology Corp, Rockingham, VT). Digital images are acquired through a Retiga EXi Fast 1394 Mono Cooled digital camera (Qimaging, Burnaby, BC, Canada). The camera has 1392x1040 of 6.45 µm square pixels; thus through a 20X objective the actual imaged area is 448 µm x 335 µm. The camera is installed on a side port and receives 80% of the collected light. An X-Y-Z stage (MS-2000, Applied Scientific Instrumentation, Eugene, OR) with analog video autofocus algorithm is used to move the sample to pre-determined X,Y location and to obtain the best focal plane using a Z-drive attached to the fine focus shaft of the Nikon microscope. The autofocus feedback is enabled by a monochrome video camera (CCD100, DAGE-MTI Inc., Michigan City, IN) that senses 20% of the DAPI signal through a trinocular head and feeds the signal intensity to the MS-2000 controller for contrast comparison. The filter changer, the shutters, and the digital camera are all controlled by a commercial software package (SimplePCI+AIC, Compix Inc., Cranberry Township, PA) and the autofocus is triggered every time the stage is moved to a new location.

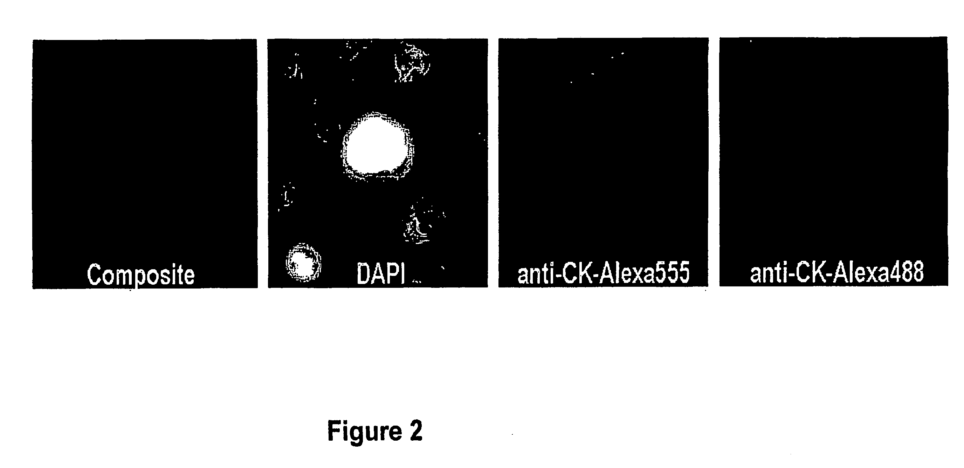

*CTC Identification.* CTCs are identified from 40X, 3-color fluorescent images that exhibit fluorescence from the two cytokeratin (CK) secondary antibodies and the DAPI nuclear stain. CTC identification is made independently by at least one pathologist, who was blind of the subject status. The CTC identification criteria consist of CK positive fluorescence with an appropriate, CK-typical staining pattern in both Alexa 555 and Alexa 488 combined with DAPI fluorescence. Typical morphology characteristics include an enlarged, round cell and nucleus, a high nuclear to cytoplasmic ratio, and striated cytokeratin fluorescence that resembles the underlying cytoskeleton. Only intact, well-defined cells are counted as CTCs (Figure 2) in this study. The false positives are primarily a result of antibody aggregates.

Figure 2 shows an example of pathologist identified CTC found in the peripheral blood of a breast cancer patient (20x). The CTC is stained with anti-CK-AlexaFluor 488 (green) and anti-CK-AlexaFluor 555 (red). The cell nuclei are stained blue with DAPI. A) composite image, B) DAPI only, C) anti-CK-AlexaFluor 555, D) anti-CK-Alexa 488.

*Wright-Giemsa Staining.* Coverslips are removed from fluorescently stained slides and rinsed in PBS. The slide is then flooded with Wright-Giemsa stain (Fisher Scientific, Kalamazoo, MI) for 3 minutes. 1.5mL of phosphate buffer pH 6.8 (Fisher Scientific, Kalamazoo, MI) is added to the stain-covered slide and the stain and buffer are mixed together by gently rocking for 1 minute. The mixture is then allowed to stand on the slide for 2 more minutes before the slide is rinsed with deionized water and allowed to air dry.

### EXAMPLE 2

### Locating Circulating epithelial Tumor Cells by FAST Technology

FAST technology has been used to rapidly locate CTCs in blood samples from Stage IV breast cancer patients. The fluorescent objects identified in the FAST scan are in turn relocated with an automated stage on a fluorescent microscope and imaged with a digital camera. High resolution images of the objects are subsequently analyzed for CTCs. The FAST location accuracy is +/-70 µm, which is more than adequate for automatic relocation in the imaging field of view (448 µm x 335 µm) with a 20X microscope. The ADM relocation of the FAST-identified objects is fully automated. Alignment marks (20µm fiducial cross-hairs visible to FAST and bright field microscopy) are used to transform the FAST-identified object positions into the microscope coordinate system. An autofocus is used to focus on each image independently using the DAPI staining of white blood cells.

The time for relocating and imaging a FAST-identified object in the ADM is about 8 seconds an object: this includes 2.5 seconds for exposure and filter switching, 1.5 seconds for stage travel and settling, 3 seconds for autofocus plus I second for other intermediate steps and writing data to networked attached storage. With a specificity of 10⁻⁵, analysis of a 60M-cell sample, which is typical of a 10 ml sample, requires image acquisition for 600 objects and takes about 80 minutes.

The repeatability of the system has been tested using a model sample of peripheral blood spiked with cells from the HT-29 colorectal cell line. Three samples were prepared each containing HT-29 cells in the range of 10 to 21 cells. Each sample was scanned 10 times with the FAST cytometer and then took high resolution images of all the objects. In each case, the FAST detected exactly the same cells in the same locations. The samples were then scanned with the microscope without using the FAST locations using cell image identification software. The microscope scanning detected all of cells that were detected by the FAST cytometer and no additional cells.

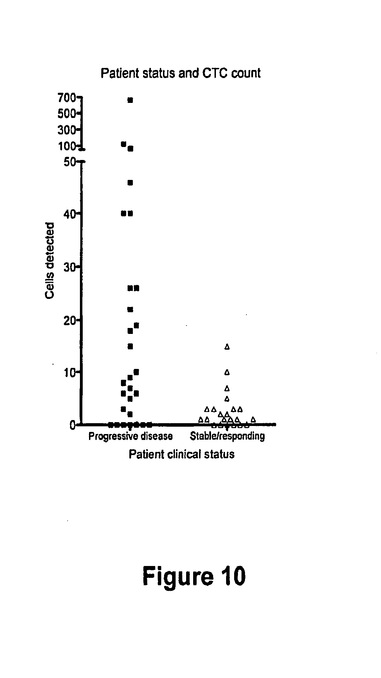

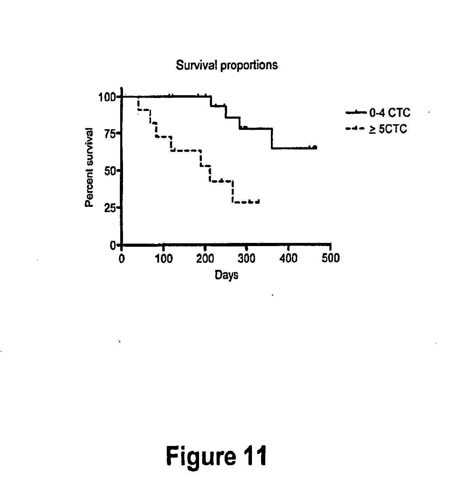

The system has been used to detect CTCs in breast cancer patients as described in the Methods section. 31 women with metastatic breast cancer were enrolled in the study and provided 50 peripheral blood specimens. Patients with progressive disease had significantly higher CTC counts (p<0.0001) than those who were stable or responding to therapy. The median CTC count for patients with progressive disease was 8.5, while the median CTC count for patients with stable or responding disease was 1 (Figure 10). At a median follow-up of 1 year, 11 patients had died. As shown by Kaplan-Meier analysis, patients with ≥5 CTCs had a median survival of 212 days (Figure 11), while the median survival for patients with 0-4 CTCs had not been reached after 1 year (p=0.0012). Peripheral blood samples from healthy donors were also scanned, and no CTCs were found in any of the 15 healthy donor samples.

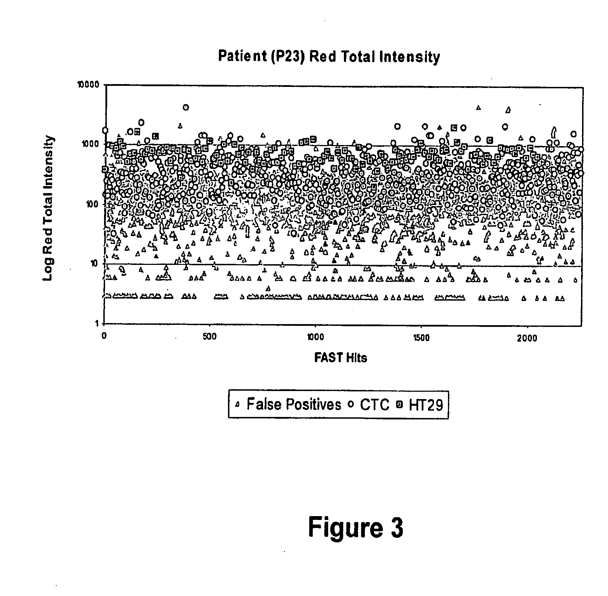

To investigate the affect of CK expression level on detection sensitivity, the brightness of CTCs was evaluated, and false positives detected in a 10 ml sample from a cancer patient with a large number of CTCs. Figure 3 shows a plot of total intensity of objects identified by FAST for a cancer patient and for HT-29 cells on a separate substrate used to monitor the sample preparation. Data with an unusually large number ofCTCs was chosen to illustrate the distribution in CTC intensity, which is typical of other samples. While the CTC intensity level spans 2 logs, all of them are at least 10 times brighter than the detection limit of FAST, as demonstrated from detection of dim false positives. This provides assurance that CTCs are being detected even with such a large range of fluorescent levels. Also shown is the fluorescence intensity from HT-29 cells processed along with the patient sample on a separate substrate. Variation of intensity of HT-29 cells is substantially less than the variation in the CTC intensities. This difference suggests there is a large variation in CK expression in CTCs.

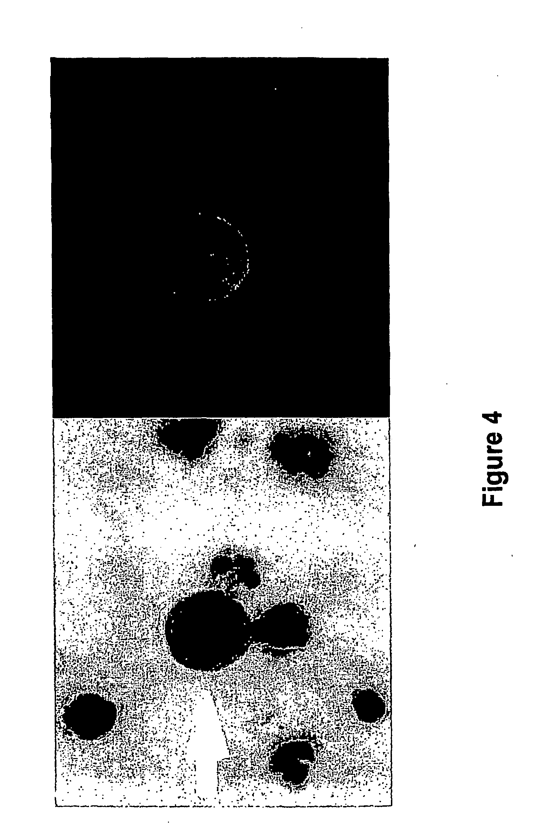

Although fluorescent images of CTCs have aided in their identification, a standard Wright-Giemsa pathology stain was implemented to provide additional validation and information about CTCs. To do this staining, the protocol was extended to remove the coverslip on each fluorescently stained slide and re-stain the same cells using Wright-Giemsa staining. B.F. Atkinson, Atlas of Diagnostic Cytopathology. 2nd Edition, W.B. Saunders Company, Ed., 2003. This staining enables improved analysis of morphology, size, and heterogeneity. Since the CTC location is accurately known, individual rare cells and rare cell clusters can be located and re-imaged with ADM for morphologic evaluation as illustrated in Figure 4.

Figure 4 shows Wright-Giemsa stain (Left) of typical CTC found in breast cancer patients (100x oil); Fluorescent image (Right) of corresponding CTC stained with anti-CK-AlexaFluor 488 and anti-CK-AlexaFluor 555 (red). The cell nuclei are stained blue DAPI (image taken at 20x and enlarged for comparison purposes).

### EXAMPLE 3

### Optimization of Circulating epithelial Tumor Detection and FAST Image Analysis

Automated digital microscopy imaging takes about 8 seconds per fluorescent object located by FAST, which results in an 80 minute per patient image acquisition time at the current specificity. While this acquisition time is adequate for research, faster acquisition would be valuable for clinical laboratory applications. To increase the speed of CTC detection, two approaches are explored to reduce the data acquisition time: improving the specificity and reducing the microscope scan time.

A major increase in specificity is expected to result from improved FAST image analysis. Label ratio, fluorescence intensity, and object size can be used to differentiate CTCs from false positives. To optimize the image analysis filters, a database of FAST-scan characteristics for true and false positives will be built. With this database the FAST software filters can be optimized without reducing sensitivity. Improvements will be made in the sample preparation process to reduce false positives. Such process changes include observing strict reagent preparation and preservation protocol, optimizing dye concentration to minimize dye aggregates and alternative blocking to minimize non-specific binding. In addition, automating the sample preparation is expected to result in a consistent reduction of fluorescent artifacts. More than 2-times improvement in specificity is expected through improved filtering and sample preparation.

To reduce the ADM image acquisition time, several improvements will be made to reduce the times for exposure, stage travel and automated focus. The primary contributions to reducing the exposure time come from increasing the ADM sensitivity. For this, the microscope objective numerical aperture will be increased (e.g. for 20X, from 0.45 to 0.75) by reducing the working distance. A custom filter set will be designed to maximize the excitation intensity and transmission of weak fluorescence, and the mercury light source will be replaced with a brighter one, such as a xenon lamp. With the relocation accuracy, the CCD pixels can be binned to reduce exposure times. For example, a 2x2 binning reduces the image size to 224x117 µm, which is large enough to capture all FAST-identified objects. These improvements are expected to reduce the exposure time 4-fold, from 2s to 0.5s.

To improve the automated focus, the sole mechanical z-motion will be replaced with a combination of fine-scale motion implemented with a piezo actuator in the stage and a course-scale mechanical motion. This improvement will significantly reduce the focusing time (from ~1s s to 15 ms) by eliminating the mechanical turning of the servo motor and reducing the latency in the stage movement to start the autofocus. With all of these improvements, the image acquisition time is expected to be reduced by more than 50% to less than 4 seconds, which along with improvements in specificity will reduce the average ADM image acquisition time to below 20 min for a 10 ml sample.

The FAST instrument provides detection accuracy comparable to automated digital microscopy. The overall detection sensitivity and specificity were demonstrated previously. Krivacic et al., Proc Natl Acad Sci U S A 101: 10501-10504, 2004. Here the instrument has been shown to have excellent measurement repeatability and a detection threshold that is more than adequate to detect large observed variations in CTC intensity. This intensity is attributed variation to variations in expression levels of CK in the CTCs. Such variations are consistent with reported low expression levels in carcinomas. Willipinski-Stapelfeldt et al., Clin Cancer Res 11: 8006-8014, 2005.

For CTC identification, several criteria are used based on cell morphology, including details of cell size, nucleus to cytoplasmic ratio, fluorescence pattern and intensity to identify cell populations that were present exclusively in breast cancer patients. Using these criteria, no cells were found in 15 healthy controls. Patients with progressive disease had a trend toward more CTCs per sample than patients with stable or responding disease.

The limited sample size, limited follow-up, and ongoing method development in this series, however, precludes any statements about the prognostic significance of FAST detected CTCs at this time. In addition since the sample sizes are small and not planned prospectively, statistical testing showing significant differences between groups does not add meaningful information to the dataset and was deliberately excluded.

The large variation in CTC levels (0-659 CTCs) that were detected in the 31 women with metastatic breast cancer reported here is similar to the range reported by Cristofanilli (0-1000s) for a much larger study of 177 patients. This large dynamic range of CTC counts in the present series and those of others is on the same - 3 log order of magnitude as observed with many commonly used protein tumor markers in plasma, and reflects the biological heterogeneity of metastatic breast cancer. However, it was found that CTCs are nearly universally present in patients with metastatic breast cancer, having detected them in 86% of metastatic patients analyzed. For comparison, Cristofanilli and colleagues reported >2 CTCs in 61% of patients with progressive metastatic breast cancer, while Allard found > 2 CTCs in 37% of a group of 422 stable and progressing metastatic breast cancer patients. Allard et al., Clin Cancer Res 10: 6897-904, 2004; Cristofanilli et al., N Engl J Med 351: 781-91, 2004. A trend for patients with progressive disease to have a higher number of CTCs was found compared with stable patients. This observation may be relevant clinically in that a high CTC count may identify those patients who are on ineffective therapy and suggest to the clinician that treatment should be modified. Current clinical practice relies on the use of imaging studies which are not available as point-of-care diagnostic tests to identify when patients have progressive disease. To determine if FAST scanning may serve as a less expensive and more convenient method for making treatment decisions, prospective evaluations of FAST as a surrogate for imaging evaluation to guide choice of chemotherapy in patients with metastatic breast cancer are required. The role of FAST in primary screening for breast cancer in unaffected women or the secondary screening of women with early stage cancer or non-metastatic disease for evidence of relapse is unknown. Additional data on the incidence of CTCs in stage I and II breast cancer patients at diagnosis and after primary therapy are required to determine the value of CTC detection as a screening methodology.

Because FAST scanning identifies cells using internal cytokeratins that are preserved even in the most undifferentiated epithelial tumors, it may have advantages over techniques that, in order to achieve enrichment, are dependent on the detection of variably expressed surface proteins that are not uniformly expressed in all patients with breast cancer.
For example, subsets of breast cancer patients may not express Ep-CAM and therefore not be detected using that method for enrichment. One such subgroup may be lobular carcinoma patients. This subset comprises 15% of breast cancer patients, and has little or no Ep-CAM expression on primary tumor specimens as measured by immunohistochemistry. Went et al., Human Pathology 35: 122-128, 2004. Additionally, Ep-CAM is known to be down-regulated in CTCs compared with the primary tumor. Rao et al., Int J Oncol 27: 49-57, 2005. The favorable results with FAST thus far do justify additional patient sampling in the context of a prospective clinical trial comparing patient outcomes in groups with different numbers of CTCs, and direct comparisons of FAST and other technologies in patient populations.

Finally, the clinical diagnosis of carcinoma in patients is substantially benefited by the ability to perform pathologic evaluation of the malignant cells using standard histologic stains. Wright-Giemsa staining was used in the present exemplary embodiments because it is the most commonly used stain on clinical peripheral blood specimens. The ability to revisit and re-stain cells with FAST makes the technology a feasible mechanism for the morphologic diagnosis of carcinoma from peripheral blood samples. This capability is essential if analysis of CTCs is to be applied to patient populations who are not otherwise known to have detectable metastatic disease, as few clinicians would trust immunofluorescent images alone to make a diagnosis of cancer. Careful cytomorphologic evaluation of intact cells by a pathologist with incorporation of ancillary evaluation techniques remains the gold standard for the diagnosis of cancer from limited numbers of malignant cells. Because breast cancer cells in blood have not been extensively studied morphologically due to their rarity and may have different morphologic features than in other body sites, an image gallery or 'atlas' of CTCs from a wide array of breast cancer patients will need to be acquired as part of the diagnostic evolution. FAST is an ideal tool for creating an 'atlas' of such images.

It has been demonstrated that the FAST scanning can quickly and accurately locate CTCs at very low concentrations in peripheral blood. The instrument has excellent repeatability and has a more than adequate detection threshold for detecting cells with large variations in expression levels of the target antigen, CK. This scan system was integrated with ADM and used this system to identify CTCs in most stage IV breast cancer patients while detecting no CTCs in 15 healthy donors, which compares favorably with other reported studies. Furthermore, one can relocate and examine cells of interest for pathologic confirmation and characterization using the Wright-Giemsa stain. This analysis of CTCs will be extended using additional stains and cancer cell biomarkers to further characterize CTCs and establish a greater clinical significance.

This system promises to enable new research into the morphological classification molecular characterization of CTCs as well as applications for point-of-care screening, monitoring and management of cancer patients.

### EXAMPLE 4

### CTC Counts in a Subset of Stage IV Metastatic Breast Cancer Patients

CK positive CTCs were examined in over 30 patients with known metastatic breast cancer. The cells were identified using FAST scanning with subsequent fluorescent microscopy as described above. A blinded review by a pathologist confirmed the identity of CTCs in these patients from 40X, 3-color fluorescent images. Criteria for CTC identification from fluorescent images consists of CK positive fluorescence in both Alexa 555 and Alexa 488, with an appropriate CK-typical staining pattern, overlying the DAPI nuclear stain. In the initial validation study outpatient stage III and stage IV metastatic breast cancer patients with varying degrees of disease was examined. In all but one metastatic cancer patient CTCs were identified ranging from 1 to over 1000 CTCs. Additionally, 10 healthy donors were examined in which no CTCs were identified.

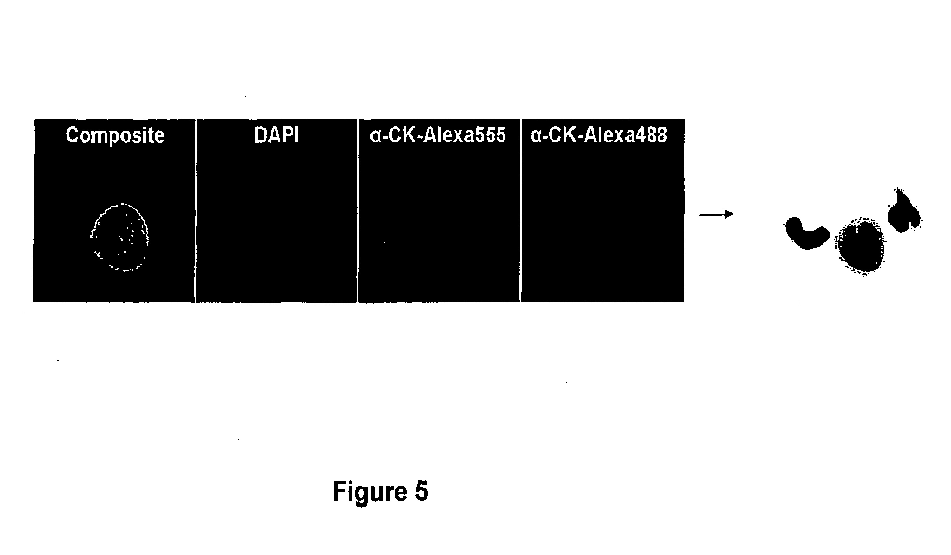

Six representative metastatic breast cancer patients were selected with varying types of primary tumor characteristics to investigate inter- and intra- patient CTC populations. A broad range of CTCs, ranging from 6 to 659 CTCs per patient, were identified using the criteria established above in Patients A through F. Clinical data on these patients is summarized in Table 1. Subsequent to acquisition of fluorescent images, a subset of identified CK positive individual rare cells and rare cell clusters from this population were relocated and further evaluated after staining with Wright-Giemsa (Figure 5).

Figure 5 shows i) Example of pathologist identified CTC found in the peripheral blood of a breast cancer patient (60x). The CTC is stained with anti-CK-AlexaFluor 488 (green) and anti-CK-AlexaFluor 555 (red). The cell nuclei are stained blue with DAPI. A) composite image, B) DAPI only, C) anti-CK-AlexaFluor 555, D) anti-CK-Alexa 488. ii) Corresponding Wright-Giemsa stain of the same CTC (100x).

**Table 1. Clinical information from subset of metastatic breast cancer patients.**

| Patient | Age | Primary Tumor Size | Primary Tumor Grade | Stage at Diagnosis | Tumor Type | Number of CTCs |
|---|---|---|---|---|---|---|
| **A** | 58 | 1.2cm | BSR 7/9 | T4N1 | infiltrating ductal | 26 |
| **B** | 66 | 0.8cm | unavailable | T1N0 | infiltrating ductal | 26 |
| **C** | 37 | 1.7cm | BSR 7/9 | T1N1 | infiltrating ductal | 659 |
| **D** | 45 | 1.0cm | BSR 6/9 | T1N0 | infiltrating ductal | 19 |
| **E** | 40 | 4.5cm | BSR 9/9 | T2N0 | infiltrating ductal | 46 |
| **F** | 49 | >2, <5cm | BSR 7/9 | T2N1 | Mixed inf. ductal/lobular | 6 |

### EXAMPLE 5

### Morphologic Characteristics of CTCs: Four Recognizable Subtypes

Because of the morphologic detail provided by the Wright-Giemsa stained cells, the morphologic criteria has been expanded from what was previously reported. Complementing the fluorescent analysis reported by Fehm et al., Wright-Giemsa stain followed by light microscopy analysis creates detailed nuclear and cytoplasmic information, highlighting features required for a morphologic cytopathologic diagnosis of carcinoma. Fehm et al., Cytotherapy 7: 171-185, 2005. As well, this enables the identification of CTCs with variable nuclear to cytoplasmic ratios, similar to those seen in primary and metastatic tumor tissue, suggesting that they be considered part of the CTC population. Fluorescent and light microscopy analysis of CTCs showed a high degree of pleomorphism in whole blood preparations, both between patients and within patients of this patient subset. Analysis of the Wright-Giemsa stained CTCs was used to classify CTCs in several morphologic subtypes, as detailed below.

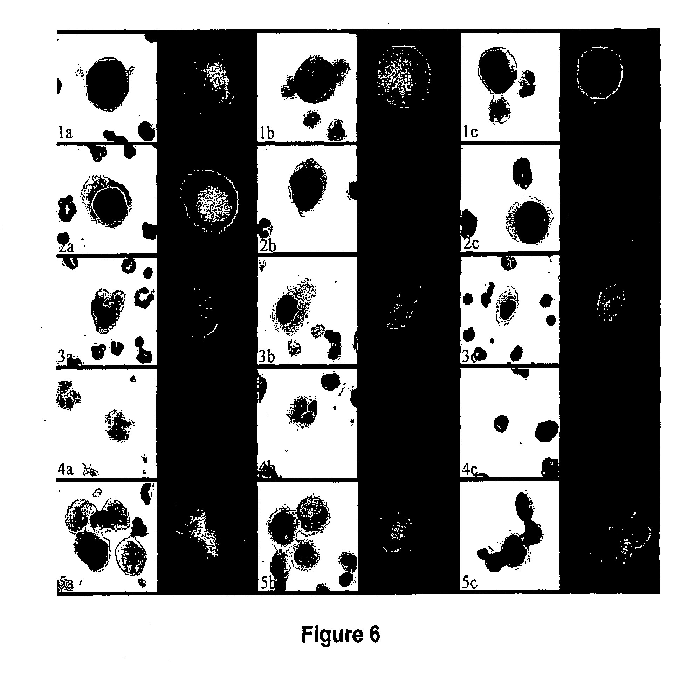

The patients' CTCs were classified into the following four morphologic types: 1) intact cells with high N/C (nuclear-to-cytoplasmic) ratios, 2) intact cells with low-to-moderate N/C ratios, 3) cells showing morphologic features of early apoptosis, and 4) cells showing morphologic features of late apoptosis. Example CTCs of these categories are displayed in Figure 6 and the proportions of CTCs found in Patients A through F using this classification is shown in Table 2. The classification categories are discussed in more detail.

Figure 6 shows an example ofCTCs identified from Patient C. Paired images of cells showing Wright-Giemsa staining result and fluorescent CK+ image. Left: Wright-Giemsa stain of CTC (100x oil). Right: Fluorescent image of corresponding CTC stained with anti-CK-AlexaFluor 488 (green). The cell nuclei are stained blue DAPI (image taken at 20x and enlarged for comparison purposes). 1a-c) high N/C type 1 cells, 2a-c) low N/C type 2 cells, 3a-c) early apoptotic type 3 cells, 4a-c) late apoptotic type 4 cells, 5a-c) clustered CTCs.

**Table 2. Percentage of CTCs identified in categories. [ ] = absolute number of cells identified in specific category. No CTC clusters were found in Patient A, E, of F. Patient B had two doublets, Patient C had seven doublets, one triplet, and one quadruplet cluster, Patient D had two doublets and a cluster of 9 CTCs.**

| **Patient** | **A** | **B** | **C** | **D** | **E** | **F** |
|---|---|---|---|---|---|---|
| **Type 1 (High N/C)** | 34.6% [9] | 30.8% [8] | 22.9% [151] | 36.8% [7] | 30.4% [14] | 16.7% [1] |
| **Type 2 (Low N/C)** | 3.8% [1] | 3.8% [1] | 10.5% [69] | 42.1% [8] | 19.6% [9] | 0.0% |
| **Type 3 (Early apoptotic)** | 57.7% [15] | 57.7% [151] | 31.9% [210] | 15.8% [3] | 26.1% [12] | 66.7% [4] |
| **Type 4 (Late Apoptotic)** | 3.8% [1] | 7.7% [2] | 34.7% [229] | 5.3% [1] | 23.9% [11] | 16.7% [1] |
| **Total Cells** | **26** | **26** | **659** | **19** | **46** | **6** |

Type I cells, or 'high N/C ratio cells', are slightly to much larger than the surrounding white blood cells, with very high nuclear-to-cytoplasmic ratios and only a scant rim of amphophilic to eosinophilic cytoplasm. The nuclei are round to oval to occasionally gently lobated, without sharp irregularities. The chromatin is often vesicular, with prominent, generally eosinophilic nucleoli. These cells conform cytomorphologically to accepted criteria for both true positive CTCs as discussed Fehm, as well as for standard cytopathologic criteria for poorly differentiated carcinoma cells. Fehm et al., Cytotherapy 7: 171-185, 2005; Atkinson, Atlas of Diagnostic Cytopathology. Edited by W.B. Saunders Company, 2003.

Type 2 cells, or 'low N/C ratio cells,' are diagnostic for metastatic carcinoma cells upon careful review of the detailed morphologic images, however, they do not meet all of the currently established criteria for CTCs. Due to small size or low N/C ratio, they would be from counts by other investigators; however, because of the correlation with the Wright-Giemsa stain, they can be classified as CTCs. The cells show intact nuclear detail, without morphologic evidence of apoptotic changes, but in contrast to type 1 cells, these cells have a lower nuclear-to-cytoplasmic ratio. Additionally, they may be slightly smaller than type 1 cells, with a diameter approximately equal to or even occasionally smaller than neighboring white blood cells, and have less prominent nucleoli. The nucleus is occasionally eccentrically located, and the cells have a moderate rim of dense appearing orangeophilic cytoplasm on Wright-Giemsa staining. This category of cell corresponds to moderately-to-well differentiated carcinoma cells. Atkinson, Atlas of Diagnostic Cytopathology. Edited by W.B. Saunders Company, 2003.

The remaining two categories of cells are those showing morphologic evidence of apoptosis and are classified according to the degree of morphologic change identified, into 'early apoptosis' and 'late apoptosis.' Atkinson, Atlas of Diagnostic Cytopathology. Edited by W.B. Saunders Company, 2003. Although an argument could be made for merging these two categories and including all cells showing any morphologic evidence of apoptosis within one large category, they are separated here for purposes of study. The two categories of morphologically apoptotic cells comprise the majority of detected CTCs in this group of stage IV patients.

Type 3, referred to here as 'early apoptosis', are cells showing the initial morphologic changes associated with early apoptosis. The cells show condensation and shrinkage of nuclear material, yielding hyperchromatic 'glassy' appearing nuclei with loss of nuclear detail on Wright-Giemsa staining, but without overall loss of contour and without nuclear fragmentation. Occasional 'nicking' of the rounded nuclear contour is classified as 'early' apoptosis, in contrast to distinct fragmentation of the nucleus, described below in the 'late' category. Alterations of cytoplasm without evidence of nuclear change is also categorized as 'early', for example, formation of cytoplasmic inclusions or 'blebbing' in a cell with otherwise intact nuclear detail. The 'early' category of cells contains many of the CTCs that seem surprisingly small in size for a carcinoma cell, and likely explains some of the size heterogeneity noted here and in other published descriptions of circulating populations as many of the observed cells are in the process of dying. Mehes et al., Haemalologia (Budap) 31: 97-109, 2001.

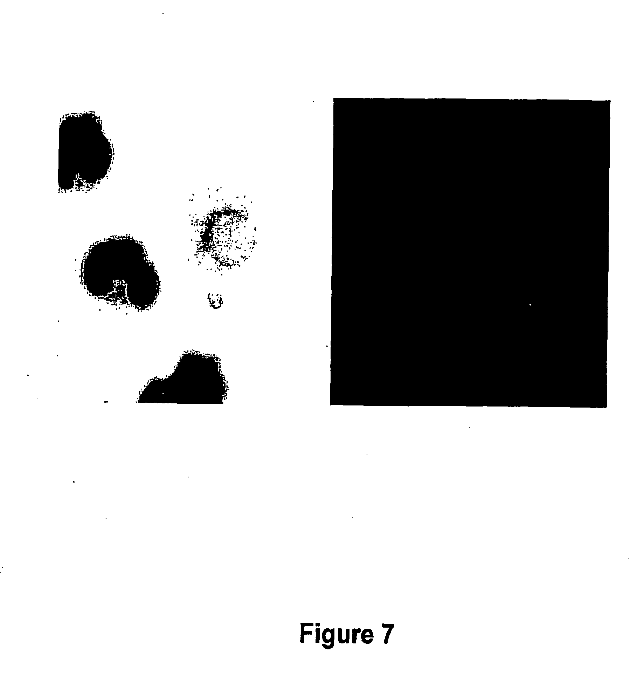

Type 4 cells, referred to here as 'late apoptosis' show well-developed morphologic features ofapoptosis, such as nuclear fragmentation. Included in this category are cells with the overall cytoplasmic contour of a typical carcinoma cell, but with loss of nuclear material to the extent that the only remaining nuclear fragment may be a tiny blue blob on Wright-Giemsa staining. Schmidt et al., Int J Biol Markers 19: 93-99, 2004. Initially, some of these cells were considered as possible false positives in the present fluorescent detection system, but careful review of their cytomorphology with Wright-Giemsa staining and comparison with other CTCs showing changes that fall along the spectrum from early to late to entirely degenerated, suggesting these CTCs exhibit very late apoptotic changes. In fact, occasional cell-like structures consisting of clusters of cytokeratin positive inclusions with Wright-Giemsa characteristics of cytokeratin-laden cytoplasm (dense orangeophilic cytoplasm) likely represent the actual final stage of apoptosis, but due to the complete absence of any nuclear material within these structures, they are not counted in the present system as CTCs (Figure 7). Figure 7 shows a probable CTC undergoing final stage of apoptosis. Cells like this are not used in CTC counts, as the nuclear material is absent.

Finally, two other morphologic variants are noted with some frequency, which, although not felt to merit a separate morphologic category, nonetheless may yield information relevant to CTC biologic behavior. The first of these is clusters, including doublets, triplets and occasional clusters of five to nine cells (Figure 6. See 5a-5c.). CTC clusters make up one or more of the first 4 categories mentioned above and each cell is counted as a separate CTC. The second consists of small, apoptotic (type 3 or 4) cells that are in the process of being actively engulfed by white blood cells with nuclear features of monocytes. Whether this phenomenon represents a frequent, directed mechanism of clearance of CTCs by the circulating component of the reticuloendothelial system, or is a random nonspecific event is unclear.

### EXAMPLE 6

### Case Study of Patient C: Comparison of Tumor Cells in Various Body Sites

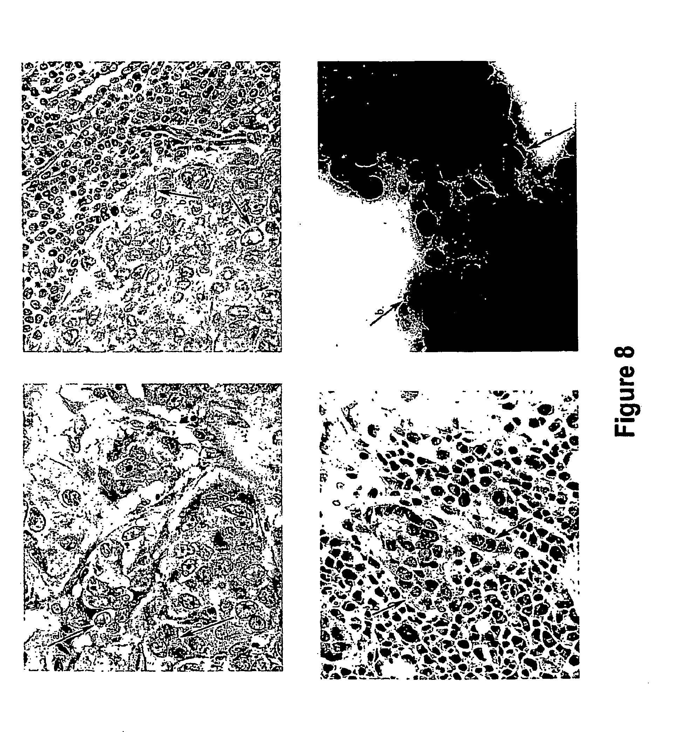

In order to understand the morphologic relationship between primary and metastatic tumor sites, and CTCs, Patient C was retrospectively studied in greater detail, including histopathologic review of the diagnostic needle biopsy of the breast, the lumpectomy and axiliary dissection, a subsequent positive bone marrow biopsy (Figure 8). Her primary tumor was a 1.7cm infiltrating ductal carcinoma, BSR 7/9 (tubule formation 3, nuclear grade 3, mitotic activity 1) with four of twenty axillary lymph nodes positive at the time of initial resection. The tumor is ER/PR positive/positive and Her-2/neu negative.

Figures 8A-8D show histologic and cytologic features of patient C's primary and metastatic tumor. Figure 8A shows a primary tumor from patient C, showing infiltrating ductal carcinoma. The malignant nests show pleomorphic cells, some with high N/C ratios (arrow a) and some with low N/C ratios (arrow b.) II shows an H&E stained section of a lymph node metastasis from patient C, showing variability in N/C ratio within the metastasis. a) Metastatic tumor cell with a high N/C ratio. b) Metastatic tumor cell with a low N/C ratio. Figure 8C shows a PAS stained core biopsy section from patient C, showing pleomorphism within bone marrow metastasis. a) High N/C ratio cell. b) Low N/C ratio cell. Figure 8D shows Wright-Giemsa stained bone marrow aspirate monolayer from patient C. a) High N/C ratio cells. b) Low N/C ratio cell.

Cytomorphologic comparison between the tumor cells in the primary and in the various metastatic sites demonstrates cytologic concordance; thus, in this case, as in many metastatic breast cancer cases, there is no evidence of significant dedifferentiation in metastatic sites. Furthermore, comparing the intact CTCs (Table 2, Type 1 and 2) to the primary and metastatic tumor cells shows a mixture of high N/C ratio cells and low N/C ratio cells in all sites. This correlation demonstrates preservation of the inherent pleomorphism of this patient's tumor in various different sites, with no particular morphologic subcategory of cells, i.e. large high N/C ratio cells, predominating in one site versus another. Thus, evidence to suggest homing of a morphologically distinct subclone of cells to the peripheral blood as compared to other sites is not identified.

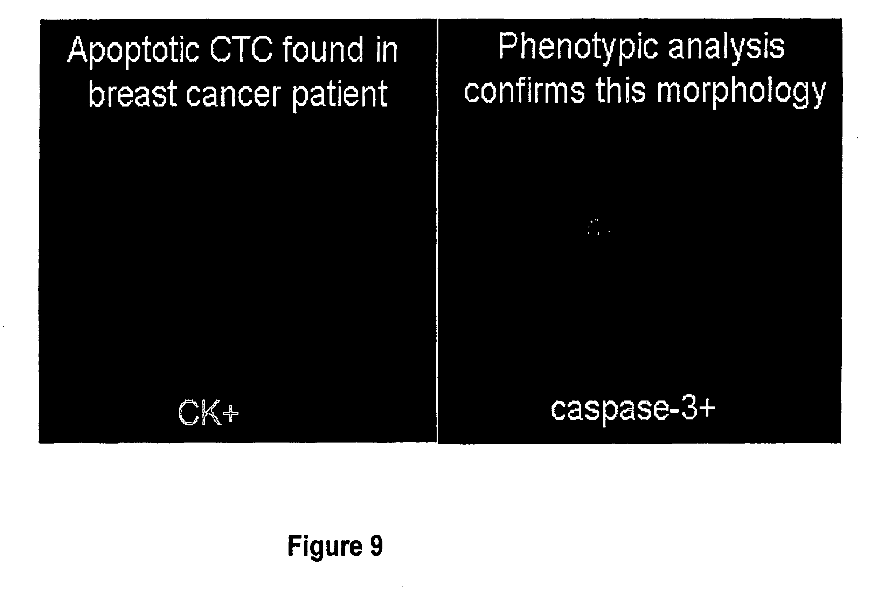

Figure 9 shows a CTC found in a breast cancer patient that was morphologically described as undergoing apoptosis. The paired image is positive for cytokeratin (fluorescent CK⁺) and fluorescent caspase-3⁺. Phenotypic analysis confirms that the cell is apoptotic.

### EXAMPLE 7

### Images of Circulating epithelial Tumor Cell From Patients Describe Morphology of a Distinct Cell Population

Due to their rarity, breast cancer CTCs are only beginning to be extensively morphologically studied. The protocol and technology of the present invention enables one to create a gallery of CTC images stained with a standard pathology stain from a variety of patients to begin to describe the morphology of CTCs as a distinct cell population in a newly accessible tissue compartment.

As used in standard pathology practice, stains such as Wright-Giemsa yield information on nuclear detail, nuclear contour, presence or absence of nucleoli, quality of cytoplasm, and amount of cytoplasm, and allow for distinction between a malignant tumor cell and benign background cells in tissue samples. The use of the Wright-Giemsa stain was implemented to enable a morphologic comparison of CTCs to tumor cells recovered from other tumor sites.

The current classification of CTCs using fluorescence and Wright-Giemsa staining is in general agreement with other reports of the morphologic appearance of CTCs but expands the previous CTC classification categories by proposing the presence of a subpopulation of smaller, lower N/C ratio cells among the circulating population. Fehm et al., Cytotherapy 7: 171-185, 2005. Additionally, the presence of apoptotic cells were morphologically confirmed, as has been reported by previous investigators, and this population was further subclassified into cells showing early and late changes. Larson et al., Cytometry A 62: 46-53, 2004; Mehes et al., Am J Pathol 159: 17-20, 2001.

Clusters of CTCs are also common in some patients, and have been described in previous papers as pathognomic for CTCs as well. Fehm et al., Clin Cancer Res 8: 2073-2084, 2002. In standard pathology practice, when evaluating other semisolid or liquid tissues, such as pleural or ascitic fluid and bone marrow aspirate material, the presence of clusters has been accepted as strong evidence of epithelial differentiation, and thus of malignancy in sites where epithelial cells are normally not found.

Breast cancers are known to display a heterogeneous phenotype, both in original tumors and in metastatic foci. Klein et al., Lancet 360: 683-689, 2002; Kuukasjarvi et al., Cancer Res 57: 1597-1604, 1997. One question is whether the cells in transit between these two locations display a similar heterogeneity, or if only a morphologic subset such as the very undifferentiated, high N/C ratio cells enter the bloodstream. Although the question of which fraction of CTCs actually have the biologic potential to form metastatic tumors remains unanswered, the present morphologic studies of CTCs suggests that the population that needs to be considered as possible suspects includes the entire spectrum of cells within a tumor, as all are found circulating in the blood. This observation is in line with the observations from Klein and colleagues who demonstrated cytogenetic evidence that the selection of clonally expanding cells leading to metastasis occurs after dissemination has taken place. Klein et al., Lancet 360: 683-689, 2002. While some CTCs found in breast cancer patients are large, round, have a high nuclear to cytoplasmic ratio, and have moderate to high expression of CK, many do not display one or more of these features. The heterogeneity of cells between Patients A-F and within Patient C alone illustrates CTC pleomorphism both among and within patient samples.

Patient C, who was selected for extensive morphologic correlation, has high numbers of CTCs. The cytologic composition of cells in the patient's primary tumor, axillary metastases, bone marrow metastases, and peripheral blood is similar, with the exception that more apoptotic forms are noted in the peripheral blood. Thus, in this patient there is no morphologic evidence that the circulating component represents a particularly poorly differentiated subclone, but rather appears representative of the phenotypically heterogeneous tumor cell population in the primary tumor.

The cell attachment protocol in tandem with FAST scanning allows a clinician or pathologist to combine a sensitive and specific detection method with sequential analysis of CTCs, and importantly, provides for relocation of the cells of interest in their fixed position for additional study, including cytomorphologic diagnosis. Including the Wright-Giemsa stain into the assay has enabled the inclusion of standard cytopathologic methods in classifying the types of CTCs that are found in the peripheral blood of metastatic breast cancer patients. Inter- and intra-patient CTC heterogeneity has been demonstrated. This study will be expanded with existing and newly evolving cellular biomarkers that may predict the pattern of metastatic spread in individuals, and may also be useful for identifying the tissue of origin of detected circulating epithelial cells, especially in individuals in whom cancer has not been diagnosed. The ability to interrogate CTCs with additional protein markers may allow for screening and diagnosis of epithelial malignancy from peripheral blood samples.

When ranges are used herein for physical properties, such as molecular weight, or chemical properties, such as chemical formulae, all combinations and subcombinations of ranges and specific embodiments therein are intended to be included.

Those skilled in the art will appreciate that numerous changes and modifications can be made to the embodiments of the invention and that such changes and modifications can be made without departing from the scope of the invention, as defined by the appended claims.

## Claims

1. A method for detecting circulating epithelial tumor cells in a mammalian subject suspected of having cancer comprising:
(i) mounting a test sample on a substrate, the test sample being from blood of the subject, the test sample comprising a cell population which has not been enriched for circulating tumor cells;
(ii) detecting the presence or absence of a first marker in the test sample, wherein the first marker is specific for circulating epithelial tumor cells and is a cytokeratin marker;
(iii) detecting the presence or absence of a second marker in the cells of the cell population or a subset of the cell population, wherein the second marker is cell morphology, cell size, or nuclear to cytoplasmic ratio, and is detected with a cytological stain; and
(iv) analyzing the cell population detected by the first and second markers to identify circulating epithelial tumor cells.

2. The method of claim 1 wherein the substrate is a planar substrate.

3. The method of claim 1 wherein mounting the test sample on the substrate forms a biological monolayer.

4. The method of claim 1 wherein the first marker is detected using a fluorescent label.

5. The method of claim 1 further comprising analyzing the cell population by nuclear detail, nuclear contour, presence or absence of nucleoli, quality of cytoplasm, or quantity of cytoplasm.

6. The method of claim 1 further comprising analyzing the cell population by measuring intact cells with a high nuclear to cytoplasmic ratio, intact cells with a low nuclear to cytoplasmic ratio, early apoptotic cells, or late apoptotic cells, and identifying the circulating tumor cells.

7. The method of claim 1 wherein detecting the first marker further comprises analyzing the cell population by cell attachment to the substrate, scanning the cell population on the substrate by fiber optic array, and imaging the cells by digital microscopy using relocation.

8. The method of claim 7 wherein detecting the second marker further comprises relocating epithelial cells identified by the first marker by digital microscopy.

9. A method of screening a drug candidate compound for treatment of epithelial cancer in a mammalian subject, said method comprising:
(i) mounting first and second test samples on a substrate, the first sample being a sample from blood of a subject suspected of having cancer before treatment of the subject with the drug candidate compound, and the second test sample being a sample from blood of the subject after treatment of the subject with the drug candidate compound, the test samples comprising a cell population which has not been enriched for circulating tumor cells;
(ii) detecting the presence or absence of a first marker in the test samples wherein the first marker is specific for circulating epithelial tumor cells and is a cytokeratin marker;
(iii) detecting the presence or absence of a second marker in the cells of the cell population or a subset of the cell population, wherein the second marker is cell morphology, cell size, or nuclear to cytoplasmic ratio, and is detected with a cytological stain; and
(iv) analyzing the cell population detected by the first and second markers to identify circulating epithelial tumor cells in the test samples before treatment with the drug candidate compound compared to after treatment with the drug candidate compound; wherein the presence of a decreased number of the circulating epithelial tumor cells in the sample after treatment compared to a number of the circulating epithelial tumor cells in the sample before treatment indicating effectiveness of the drug candidate compound in treating the cancer in the mammalian subject.

## Patentansprüche

1. Verfahren zum Nachweisen von zirkulierenden epithelialen Tumorzellen in einem Säuger-Subjekt mit dem Verdacht, Krebs zu haben, umfassend:
(i) Anbringen einer Testprobe auf einem Substrat, wobei die Testprobe aus Blut des Subjekts stammt, wobei die Testprobe eine Zellpopulation umfasst, die nicht auf zirkulierende Tumorzellen angereichert worden ist;
(ii) Nachweisen des Vorhandenseins oder Fehlens eines ersten Markers in der Testprobe, wobei der erste Marker für zirkulierende epitheliale Tumorzellen spezifisch ist und ein Zytokeratin-Marker ist;
(iii) Nachweisen des Vorhandenseins oder Fehlens eines zweiten Markers in den Zellen der Zellpopulation oder einer Untermenge der Zellpopulation, wobei der zweite Marker Zellmorphologie, Zellgröße oder nukleär-zuzytoplasmatisch-Verhältnis ist und durch eine zytologische Färbung nachgewiesen wird; und
(iv) Analysieren der durch den ersten und den zweiten Marker nachgewiesenen Zellpopulation, um zirkulierende epitheliale Tumorzellen zu identifizieren.

2. Verfahren gemäß Anspruch 1, wobei das Substrat ein planares Substrat ist.

3. Verfahren gemäß Anspruch 1, wobei das Anbringen der Testprobe auf dem Substrat eine biologische Monoschicht bildet.

4. Verfahren gemäß Anspruch 1, wobei der erste Marker unter Verwendung einer Fluoreszenzmarkierung nachgewiesen wird.

5. Verfahren gemäß Anspruch 1, ferner umfassend das Analysieren der Zellpopulation durch nukleäres Detail, nukleäre Kontur, Vorhandensein oder Fehlen von Nukleolen, Qualität des Zytoplasmas oder Quantität des Zytoplasmas.

6. Verfahren gemäß Anspruch 1, ferner umfassend das Analysieren der Zellpopulation durch Messen von intakten Zellen mit einem hohen nukleär-zuzytoplasmatisch-Verhältnis, intakten Zellen mit einem niedrigen nukleär-zu-zytoplasmatisch-Verhältnis, früh apoptotischen Zellen oder spät apoptotischen Zellen und Identifizieren der zirkulierenden Tumorzellen.

7. Verfahren gemäß Anspruch 1, wobei das Nachweisen des ersten Markers ferner Analysieren der Zellpopulation durch Zellanhaftung an dem Substrat, Rastern der Zellpopulation auf dem Substrat durch ein faseroptisches Feld und Abbilden der Zellen durch Digitalmikroskopie unter Verwendung von Umplatzierung umfasst.

8. Verfahren gemäß Anspruch 7, wobei das Nachweisen des zweiten Markers ferner Umplatzierung von epithelialen Zellen, die durch den ersten Marker durch Digitalmikroskopie identifiziert wurden, umfasst.

9. Verfahren zum Screening einer Arzneimittelkandidatenverbindung für die Behandlung von epithelialem Krebs in einem Säuger-Subjekt, wobei das Verfahren umfasst:
(i) Anbringen einer ersten und einer zweiten Testprobe auf einem Substrat, wobei die erste Probe eine Probe aus Blut eines Subjekts mit dem Verdacht, Krebs zu haben, vor Behandlung des Subjekts mit der Arzneimittelkandidatenverbindung ist und die zweite Testprobe eine Probe aus Blut des Subjekts nach Behandlung des Subjekts mit der Arzneimittelkandidatenverbindung ist, wobei die Testproben eine Zellpopulation umfassen, die nicht auf zirkulierende Tumorzellen angereichert worden ist;
(ii) Nachweisen des Vorhandenseins oder Fehlens eines ersten Markers in den Testproben, wobei der erste Marker für zirkulierende epitheliale Tumorzellen spezifisch ist und ein Zytokeratin-Marker ist;
(iii) Nachweisen des Vorhandenseins oder Fehlens eines zweiten Markers in den Zellen der Zellpopulation oder einer Untermenge der Zellpopulation, wobei der zweite Marker Zellmorphologie, Zellgröße oder nukleär-zuzytoplasmatisch-Verhältnis ist und durch eine zytologische Färbung nachgewiesen wird; und
(iv) Analysieren der durch den ersten und den zweiten Marker nachgewiesenen Zellpopulation, um zirkulierende epitheliale Tumorzellen in den Testproben zu identifizieren, vor Behandlung mit der Arzneimittelkandidatenverbindung im Vergleich zu nach Behandlung mit der Arzneimittelkandidatenverbindung; wobei das Vorhandensein einer verringerten Anzahl der zirkulierenden epithelialen Tumorzellen in der Probe nach Behandlung im Vergleich zu der Anzahl der zirkulierenden epithelialen Tumorzellen in der Probe vor Behandlung die Wirksamkeit der Arzneimittelkandidatenverbindung bei der Behandlung des Krebses in dem Säuger-Subjekt anzeigt.

## Revendications

1. Procédé de détection des cellules épithéliales tumorales circulantes chez un sujet mammifère suspecté d'avoir un cancer, comprenant :
(i) le montage d'un échantillon d'essai sur un substrat, l'échantillon d'essai provenant du sang du sujet, l'échantillon d'essai comprenant une population cellulaire qui n'a pas été enrichie en cellules tumorales circulantes ;
(ii) la détection de la présence ou de l'absence d'un premier marqueur dans l'échantillon d'essai, le premier marqueur étant spécifique des cellules épithéliales tumorales circulantes et étant un marqueur cytokératine ;
(iii) la détection de la présence ou de l'absence d'un deuxième marqueur dans les cellules de la population cellulaire ou d'un sous-ensemble de la population cellulaire, le deuxième marqueur étant la morphologie cellulaire, la taille cellulaire ou le rapport nucléaire/cytoplasmique, et étant détecté à l'aide d'une coloration cytologique ; et
(iv) l'analyse de la population cellulaire détectée par le premier et le deuxième marqueurs, pour identifier les cellules épithéliales tumorales circulantes.

2. Procédé de la revendication 1, dans lequel le substrat est un substrat plan.

3. Procédé de la revendication 1, dans lequel le montage de l'échantillon d'essai sur le substrat forme une monocouche biologique.

4. Procédé de la revendication 1, dans lequel le premier marqueur est détecté par utilisation d'un marqueur fluorescent.

5. Procédé de la revendication 1, comprenant en outre l'analyse de la population cellulaire par le détail nucléaire, le contour nucléaire, la présence ou l'absence de nucléoles, la qualité du cytoplasme ou la quantité du cytoplasme.

6. Procédé de la revendication 1, comprenant en outre l'analyse de la population cellulaire par mesure des cellules intactes présentant un rapport nucléaire/cytoplasmique élevé, des cellules intactes ayant un faible rapport nucléaire/cytoplasmique, des cellules apoptotique précoces ou des cellules apoptotiques tardives, et l'identification des cellules tumorales circulantes.

7. Procédé de la revendication 1, dans lequel la détection du premier marqueur comprend en outre l'analyse de la population cellulaire par la fixation des cellules au substrat, le balayage de la population cellulaire sur le substrat par un réseau à fibres optiques, et l'imagerie des cellules par une microscopie numérique utilisant une translation.

8. Procédé de la revendication 7, dans lequel la détection du deuxième marqueur comprend en outre la translation, par microscopie numérique, des cellules épithéliales identifiées par le premier marqueur.

9. Procédé de criblage d'un composé candidat médicamenteux pour le traitement d'un cancer épithélial chez un sujet mammifère, ledit procédé comprenant :
(i) le montage d'un premier et d'un deuxième échantillons d'essai sur un substrat, le premier échantillon étant un échantillon provenant du sang d'un sujet suspecté d'avoir un cancer avant traitement du sujet avec le composé candidat médicamenteux, et le deuxième échantillon d'essai étant un échantillon provenant du sang du sujet après traitement du sujet avec le composé candidat médicamenteux, les échantillons d'essai comprenant une population cellulaire qui n'a pas été enrichie en cellules tumorales circulantes ;
(ii) la détection de la présence ou de l'absence d'un premier marqueur dans les échantillons d'essai, le premier marqueur étant spécifique des cellules épithéliales tumorales circulantes et étant un marqueur cytokératine ;
(iii) la détection de la présence ou de l'absence d'un deuxième marqueur dans les cellules de la population cellulaire ou d'un sous-ensemble de la population cellulaire, le deuxième marqueur étant la morphologie cellulaire, la taille cellulaire, ou le rapport nucléaire/cytoplasmique, et étant détecté par une coloration cytologique ; et
(iv) l'analyse de la population cellulaire détectée par le premier et le deuxième marqueurs pour identifier les cellules épithéliales tumorales circulantes dans les échantillons d'essai avant traitement par le composé candidat médicamenteux, par comparaison avec ces mêmes cellules après traitement avec le composé candidat médicamenteux ; la présence d'un nombre réduit de cellules épithéliales tumorales circulantes dans l'échantillon après traitement, par comparaison avec le nombre de cellules épithéliales tumorales circulantes dans l'échantillon avant traitement, indiquant l'efficacité du composé candidat médicamenteux dans le traitement du cancer chez le sujet mammifère.
